(19) Europäisches Patentamt / European Patent Office / Office européen des brevets

(11) **EP 4 480 936 A1**

(12) **EUROPEAN PATENT APPLICATION**
published in accordance with Art. 153(4) EPC

(43) Date of publication:
25.12.2024 Bulletin 2024/52

(51) International Patent Classification (IPC):
$C04B\ 35/488^{(2006.01)}$    $A61C\ 5/77^{(2017.01)}$
$A61C\ 13/083^{(2006.01)}$    $A61K\ 6/78^{(2020.01)}$
$A61K\ 6/813^{(2020.01)}$    $A61K\ 6/818^{(2020.01)}$
$A61K\ 6/822^{(2020.01)}$

(21) Application number: 23756451.3

(52) Cooperative Patent Classification (CPC):
A61C 5/77; A61C 13/083; A61K 6/78; A61K 6/813;
A61K 6/818; A61K 6/822; C04B 35/488

(22) Date of filing: 16.02.2023

(86) International application number:
PCT/JP2023/005497

(87) International publication number:
WO 2023/157926 (24.08.2023 Gazette 2023/34)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 18.02.2022 JP 2022023814

(71) Applicant: Tosoh Corporation
Yamaguchi 746-8501 (JP)

(72) Inventors:
• USHIO, Yuki
Shunan-shi
Yamaguchi 746-8051 (JP)
• AZECHI, Sho
Shunan-shi
Yamaguchi 746-8051 (JP)
• ITO, Akiko
Shunan-shi
Yamaguchi 746-8051 (JP)
• NAGAYAMA, Hitoshi
Shunan-shi
Yamaguchi 746-8051 (JP)

(74) Representative: Müller-Boré & Partner
Patentanwälte PartG mbB
Friedenheimer Brücke 21
80639 München (DE)

(54) **ZIRCONIA LAMINATE**

(57) There is provided a layered body that has gradations in translucency and color allowing the layered body to give an impression similar to that of natural teeth and with which the chipping of the surface layer (layer closer to the incisal edge of the dental restoration) can be reduced.

The layered body comprises a surface layer containing zirconia containing at least one stabilizing element and a composition-gradient layer composed of two or more unit layers. The unit layers each contain at least one coloring element and zirconia containing at least one stabilizing element, and the composition-gradient layer is constructed as a result of the unit layers being stacked in such a manner that the amount of the stabilizing element in the zirconia containing at least one stabilizing element contained in the composition-gradient layer remains unchanged or decreases from the surface layer toward the surface of the layered body opposite the surface layer. The amount of the stabilizing element in the zirconia containing at least one stabilizing element contained in the surface layer is smaller than the amount of the stabilizing element in the zirconia containing at least one stabilizing element contained in a first composition-gradient layer, which is one of the unit layers constituting the composition-gradient layer and is adjacent to the surface layer.

EP 4 480 936 A1

FIG. 1

## Description

Technical Field

The present disclosure relates to a composition in which layers of zirconia are stacked together, and also to a zirconia layered body.

Background Art

A sintered body of zirconia ($ZrO_2$) is manufactured by forming, calcining and sintering a raw material powder primarily containing zirconia. The raw material powder undergoes thermal shrinkage and densification through heat treatments such as sintering and calcination, and its behavior during the heat treatments varies depending on the characteristics of the raw material powder, particularly the composition of the raw material powder.

A large portion of the raw material powder is zirconia. Nevertheless, the thermal shrinkage behavior greatly differs even between two raw material powders that vary only in the amounts of additives constituting less than 0.1% by mass. When a green body formed by stacking layers of raw material powders with such minor compositional differences is subjected to heat treatment, defects such as partial detachment of layers or the formation of strains occur. These defects occur even with a single type of zirconia or with additives added to it. Heat treatment of green bodies without causing such defects has required special adjustments and treatments (e.g., PTL 1 and 2).

In PTL 1, it is disclosed that by adjusting the composition and thermal shrinkage behavior of raw material powders through coating with a dopant and shaping the powders, a sintered body made from a layered body with different colors can be obtained. In PTL 2, furthermore, it is disclosed that by stacking layers of powders while vibrating them to create a boundary layer as a mixture of the powders in the upper and lower layers and then shaping the stack, a sintered body made from a layered body composed of layers with different amounts of coloring elements and having variations in color can be obtained.

In the layered bodies disclosed in PTL 1 and 2, however, the zirconia constituting a large portion of the raw material powders is consistent in composition. These layered bodies are also uniform in texture, which is derived primarily from the translucent appearance of zirconia. These layered bodies, therefore, are ones that present a texture different from that of natural teeth, which have a texture derived from variations in translucent appearance.

For this reason, there is a need for a layered body of zirconia having gradations in translucency and color that allow the layered body to give an impression similar to natural teeth.

In PTL 3, there is disclosed a dental prosthetic component having gradations in translucency and color, in which zirconia composition layers containing different amounts of yttria between adjacent layers are stacked together.

Citation List

Patent Literature

PTL 1: U.S. Patent Application Publication No. 2016/0157971
PTL 2: U.S. Patent Application Publication No. 2014/0328746
PTL 3: U.S. Patent Application Publication No. 2013/0221554

Summary of Invention

Technical Problem

Known layered bodies of zirconia composition layers used as dental prosthetic components have a structure in which layers with different compositions are stacked in such a manner that the color and transmittance gradually change from the back layer (layer corresponding to the cervical region) toward the surface layer (layer corresponding to the incisal edge), with the aim of the layered bodies giving an impression similar to that of natural teeth. When layers with different compositions are stacked in such a manner that the color and transmittance gradually change, however, the surface layer (layer closer to the incisal edge of the dental restoration) exhibits high transmittance but has low mechanical strength, posing the problem that the occurrence of chipping cannot be reduced.

An object of the present disclosure is to provide at least one of a layered body having gradations in translucency and color that allow the layered body to give an impression similar to natural teeth when visually perceived and with which the chipping of the surface layer (layer closer to the incisal edge of the dental restoration) can be reduced, a precursor to it or methods for manufacturing them. From another perspective, an object of the present disclosure is to provide at least one of a layered body suitable for use as a prosthetic component for dentistry, a precursor to it or methods for manufacturing them.

Solution to Problem

The inventors noticed that by specifying the relationship between the surface layer and the amount of stabilizing element(s) in zirconia in the layer adjacent to the surface layer, the above problem can be solved. Based on this, the inventors completed the invention according to the present disclosure.

That is, the present invention is as described in the claims, and the gist of the present disclosure is as follows.

[1] A layered body comprising a surface layer containing zirconia containing at least one stabilizing element and a composition-gradient layer composed of two or more unit layers,

the unit layers each containing at least one coloring element and zirconia containing at least one stabilizing element, and
the composition-gradient layer being constructed as a result of the unit layers being stacked in such a manner that an amount of the stabilizing element in the zirconia containing at least one stabilizing element contained in the composition-gradient layer remains unchanged or decreases from the surface layer toward a surface of the layered body opposite the surface layer, wherein:
an amount of the stabilizing element in the zirconia containing at least one stabilizing element contained in the surface layer is smaller than an amount of the stabilizing element in the zirconia containing at least one stabilizing element contained in a first composition-gradient layer, which is one of the unit layers constituting the composition-gradient layer and is adjacent to the surface layer.

[2] The layered body according to [1], wherein the surface layer further contains at least one coloring element.
[3] The layered body according to [1] or [2], wherein the amount of the stabilizing element in the zirconia containing at least one stabilizing element contained in the surface layer is 2.5 mol% or more and 6.0 mol% or less.
[4] The layered body according to any of [1] to [3], wherein a difference between a stabilizing element content of the surface layer and a stabilizing element content of the first composition-gradient layer is 0.2 mol% or more.
[5] The layered body according to any of [1] to [4], wherein the amount of the stabilizing element in the zirconia containing at least one stabilizing element contained in the first composition-gradient layer is 2.5 mol% or more and 6.0 mol% or less.
[6] The layered body according to any of [1] to [5], wherein the stabilizing element is one or more selected from the group of yttrium (Y), calcium (Ca), magnesium (Mg), cerium (Ce), praseodymium (Pr), neodymium (Nd), terbium (Tb), erbium (Er) and ytterbium (Yb).
[7] The layered body according to any of [1] to [6], wherein the coloring element is at least one transition metal element, at least one lanthanoid-series rare earth element or both.
[8] The layered body according to any of [1] to [7], wherein an amount of the coloring element is 0.01% by mass or more and 1.0% by mass or less.
[9] The layered body according to any of [1] to [8], wherein warpage as measured using thickness gauges conforming to JIS B 7524: 2008 is 1.0 mm or less.
[10] The layered body according to any of [1] to [9], wherein the layered body is a sintered body.
[11] The layered body according to [10], wherein three-point flexural strength of the surface layer as measured by a method according to JIS R 1601 is 700 MPa or more.
[12] The layered body according to any of [1] to [9], wherein the layered body is a calcined body.
[13] A method for manufacturing the layered body according to any of [1] to [11], the method comprising a step of sintering a green body at 1200°C or above and 1600°C or below,

the green body having a surface powder composition layer containing zirconia containing at least one stabilizing element and a composition-gradient powder composition layer composed of two or more unit powder composition layers,
the unit powder composition layers each containing at least one coloring element and zirconia containing at least one stabilizing element,
the composition-gradient powder composition layer being constructed as a result of the unit powder composition layers being stacked in such a manner that an amount of the stabilizing element in the zirconia containing at least one stabilizing element contained in the composition-gradient powder composition layer remains unchanged or decreases from the surface powder composition layer toward a surface powder composition of the green body opposite the surface powder composition layer, and

an amount of the stabilizing element in the zirconia containing at least one stabilizing element contained in the surface powder composition layer being smaller than an amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

[14] A method for manufacturing the layered body according to any of [1] to [11], the method comprising a step of calcining a green body at 800°C or above and below 1200°C to turn the green body into a calcined body and

a step of sintering the calcined body at 1200°C or above and 1600°C or below,
the green body having a surface powder composition layer containing zirconia containing at least one stabilizing element and a composition-gradient powder composition layer composed of two or more unit powder composition layers,
the unit powder composition layers each containing at least one coloring element and zirconia containing at least one stabilizing element,
the composition-gradient powder composition layer being constructed as a result of the unit powder composition layers being stacked in such a manner that an amount of the stabilizing element in the zirconia containing at least one stabilizing element contained in the composition-gradient powder composition layer remains unchanged or decreases from the surface powder composition layer toward a surface powder composition of the green body opposite the surface powder composition layer, and
an amount of the stabilizing element in the zirconia containing at least one stabilizing element contained in the surface powder composition layer being smaller than an amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

[15] A method for manufacturing the layered body according to any of [1] to [9] or [12], the method comprising a step of calcining a green body at 800°C or above and below 1200°C,

the green body having a surface powder composition layer containing zirconia containing at least one stabilizing element and a composition-gradient powder composition layer composed of two or more unit powder composition layers,
the unit powder composition layers each containing at least one coloring element and zirconia containing at least one stabilizing element,
the composition-gradient powder composition layer being constructed as a result of the unit powder composition layers being stacked in such a manner that an amount of the stabilizing element in the zirconia containing at least one stabilizing element contained in the composition-gradient powder composition layer remains unchanged or decreases from the surface powder composition layer toward a surface powder composition of the green body opposite the surface powder composition layer, and
an amount of the stabilizing element in the zirconia containing at least one stabilizing element contained in the surface powder composition layer being smaller than an amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

[16] The manufacturing method according to any of [13] to [15], wherein the surface powder composition layer further contains at least one coloring element.
[17] The manufacturing method according to any of [13] to [16], wherein powder compositions contained in the powder composition layers are powders in a granulated state.
[18] A dental material comprising the layered body according to any of [1] to [12].

Advantageous Effects of Invention

According to the present disclosure, there can be provided at least one of a layered body that has gradations in translucency and color allowing the layered body to give an impression similar to that of natural teeth and with which the chipping of the surface layer (layer closer to the incisal edge of the dental restoration) can be reduced, a precursor to it or methods for manufacturing them. At least one of a layered body suitable for use as a prosthetic component for dentistry, a precursor to it or methods for manufacturing them, furthermore, can be provided.

Brief Description of Drawings

[Fig. 1] A schematic view illustrating a cross-section of a sintered body having a structure in which two unit zirconia layers are stacked together
[Fig. 2] A schematic view illustrating a cross-section of a sintered body having a structure in which four unit zirconia layers are stacked together
[Fig. 3] A schematic view illustrating a method for measuring warpage
[Fig. 4] A schematic view illustrating a method for measuring three-point flexural strength
[Fig. 5] A schematic view illustrating necking-structured zirconia
[Fig. 6] A schematic view describing a shaping step of uniaxial compression molding during the production of a layered body in the Examples
[Fig. 7] A schematic view describing a shaping step of CIP treatment during the production of a layered body in the Examples

Description of Embodiments

The present disclosure will now be described in detail. It should be noted that the descriptions of requirements provided below are illustrations for explaining the present disclosure; the present disclosure is not limited to what is described. The relative dimensions in the drawings, furthermore, are not limited to the proportions illustrated in the drawings.

When multiple preferred lower limits and multiple preferred upper limits are presented herein as preferred condition ranges regarding any requirement, furthermore, these lower and upper limits can be used in any combination in interpreting the preferred condition ranges.

(Layered body)

A layered body according to this embodiment is a layered body comprising a surface layer containing zirconia containing at least one stabilizing element and a composition-gradient layer composed of two or more unit layers,

the unit layers each containing at least one coloring element and zirconia containing at least one stabilizing element, and
the composition-gradient layer being constructed as a result of the unit layers being stacked in such a manner that the amount of the stabilizing element in the zirconia containing at least one stabilizing element contained in the composition-gradient layer remains unchanged or decreases from the surface layer toward the surface of the layered body opposite the surface layer.

The amount of the stabilizing element in the zirconia containing at least one stabilizing element contained in the surface layer is smaller than the amount of the stabilizing element in the zirconia containing at least one stabilizing element contained in a first composition-gradient layer, which is one of the unit layers constituting the composition-gradient layer and is adjacent to the surface layer.

The layered body according to this embodiment can be any one or more selected from the group of a sintered body, a calcined body and a green body and preferably is at least one of a sintered body or calcined body. The layered body according to this embodiment can be, for example, a sintered body when it is a dental prothesis or can be a calcined body when it is a precursor to a dental prothesis. Here, in this embodiment, "a surface layer containing zirconia containing at least one stabilizing element, unit layers containing at least one coloring element and zirconia containing at least one stabilizing element and a composition-gradient layer composed of two or more unit layers" can be changed to "a surface zirconia layer containing zirconia containing at least one stabilizing element, unit zirconia layers containing at least one coloring element and zirconia containing at least one stabilizing element and a composition-gradient zirconia layer composed of two or more unit zirconia layers," respectively, in a configuration in which the layered body is a sintered body. Likewise, in a configuration in which the layered body is a calcined body, they can be changed to "a surface zirconia composition layer containing zirconia that contains at least one stabilizing element and also has a necking structure, unit zirconia composition layers containing at least one coloring element and zirconia that contains at least one stabilizing element and also has a necking structure and a composition-gradient zirconia composition layer composed of two or more unit zirconia composition layers," and in a configuration in which the layered body is a green body, they can be changed to "a surface powder composition layer made of a powder composition containing zirconia containing at least one stabilizing element, unit powder composition layers containing a powder composition at least one coloring element and zirconia containing at least one stabilizing element, and a composition-gradient powder composition layer composed of two or more unit powder composition layers."

In the following, a layered body according to the present disclosure will be described while presenting an example of an embodiment in which the layered body is a sintered body.

(Sintered Body)

This embodiment is a sintered body having a surface zirconia layer containing zirconia containing at least one stabilizing element and a composition-gradient zirconia layer composed of two or more unit zirconia layers,

the unit zirconia layers each containing at least one coloring element and zirconia containing at least one stabilizing element, and

the composition-gradient zirconia layer being constructed as a result of the unit zirconia layers being stacked in such a manner that the amount of the stabilizing element in the zirconia containing at least one stabilizing element contained in the composition-gradient zirconia layer remains unchanged or decreases from the surface zirconia layer toward the surface of the sintered body opposite the surface zirconia layer.

The amount of the stabilizing element in the zirconia containing at least one stabilizing element contained in the surface zirconia layer is smaller than the amount of the stabilizing element in the zirconia containing at least one stabilizing element contained in a first composition-gradient zirconia layer, which is one of the unit zirconia layers constituting the composition-gradient zirconia layer and is adjacent to the surface zirconia layer.

The sintered body according to this embodiment is a composition having a multilayer structure, or is a so-called layered body, and is a layered body made of sintered tissue. In this embodiment, the sintered tissue is primarily a structure made of zirconia in its late stage of sintering.

The sintered body according to this embodiment has a surface zirconia layer containing zirconia containing at least one stabilizing element and unit zirconia layers containing at least one coloring element and zirconia containing at least one stabilizing element. Its composition-gradient zirconia layer is composed of two or more unit zirconia layers. The surface zirconia layer may contain at least one coloring element. The surface zirconia layer and the unit zirconia layers are primarily made up of crystalline particles of the zirconia containing at least one stabilizing element. The sintered body according to this embodiment, therefore, can also be considered a layered body including three or more layers containing zirconia made up of zirconia crystal particles containing at least one stabilizing element and, if it contains at least one coloring element, containing the coloring element.

<Layered body Structure>

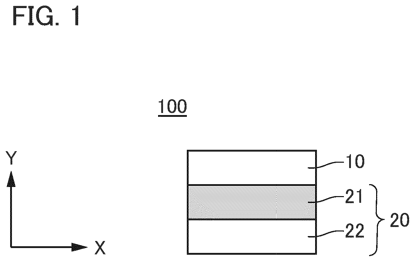

Fig. 1 is a schematic view illustrating an example of a structure of a sintered body according to this embodiment, schematically illustrating a cross-section of a sintered body (100) having a surface zirconia layer (10) and a composition-gradient zirconia layer (20) composed of two or more, multiple unit zirconia layers (two unit zirconia layers in Fig. 1). The composition-gradient zirconia layer (20) includes a first composition-gradient zirconia layer (21), which is the unit zirconia layer adjacent to the surface zirconia layer (10), and a second composition-gradient zirconia layer (22), which is the unit zirconia layer positioned at the surface of the sintered body (100) opposite the surface zirconia layer (10). The layered body (100) according to this embodiment has its surface zirconia layer (10) on one side of the first composition-gradient layer (21) and its second composition-gradient zirconia layer (22), which is a unit zirconia layer, on the other side of the first composition-gradient zirconia layer (21). In Fig. 1, the direction in which the layers are stacked together (The direction from the composition-gradient zirconia layer toward the surface zirconia layer; hereinafter also referred to as "the stacking direction.") is presented in the Y-axis direction, and the direction in which each layer extends (Hereinafter also referred to as "the horizontal direction.") is presented in the X-axis direction.

Between adjacent layers of the unit zirconia layers constituting the sintered body, the amount of the stabilizing element may be the same or different, but preferably is different. When having a structure in which zirconia layers containing zirconia with different amounts of the stabilizing element, the sintered body becomes a layered body that exhibits visible variations in color in the stacking direction based on synergistic effects, for example of the differences in the amount of the stabilizing element and translucency.

The composition-gradient zirconia layer, furthermore, is constructed as a result of the unit zirconia layers being stacked in such a manner that the amount of the stabilizing element in the zirconia containing at least one stabilizing element contained in the composition-gradient zirconia layer remains unchanged or decreases from the surface zirconia layer toward the surface of the sintered body opposite the surface zirconia layer (in the direction opposite the Y direction in Fig. 1, i.e., in the direction opposite the stacking direction). Preferably, the composition-gradient zirconia layer is constructed as a result of the unit zirconia layers being stacked in such a manner that the amount of the stabilizing element in the zirconia containing at least one stabilizing element decreases toward the back side. This allows gradations in color to be formed on the sintered body based on the synergistic effects of the differences in the amount of the stabilizing element and translucency. The amount of the stabilizing element in the zirconia containing at least one stabilizing element contained in the surface zirconia layer, furthermore, is smaller than the amount of the stabilizing element in the zirconia containing at least one stabilizing element contained in the first composition-gradient zirconia layer.

In general, when the amount of stabilizing element in zirconia contained in a zirconia layer is increased, the translucency of the zirconia layer improves, but the mechanical strength decreases. Known sintered bodies having gradations in translucency and color achieve improved translucency as a result of an increased amount of stabilizing element in zirconia in their surface layer (layer closer to the incisal edge of the dental restoration). Known sintered bodies, therefore, exhibit low mechanical strength in their surface layer (layer closer to the incisal edge of the dental restoration), preventing the reduction of the occurrence of chipping therewith. For the sintered body according to this embodiment, however, the amount of the stabilizing element in the zirconia containing at least one stabilizing element contained in its surface zirconia layer is smaller than the amount of the stabilizing element in the zirconia containing at least one stabilizing element contained in its first composition-gradient zirconia layer. In other words, for the sintered body according to this embodiment, the amount of stabilizing element in zirconia in the surface layer (layer closer to the incisal edge of the dental restoration) is small compared with that in known sintered bodies. By virtue of this, the sintered body according to this embodiment exhibits high mechanical strength in its surface layer (layer closer to the incisal edge of the dental restoration), thereby allowing the occurrence of chipping to be reduced therewith.

It should be noted that the sintered body (100) illustrated in Fig. 1 represents a state in which the layers are in contact with each other with interfaces therebetween. The sintered body according to this embodiment, however, can be in any state in which the layers have been stacked and sintered together; the layers may be stacked in a state in which they have no visible interfaces, and the interfaces between the layers are not limited to linear ones.

The sintered body according to this embodiment may have a structure in which three or more unit zirconia layers, or even four or more, are stacked together. With an increase in the number of unit zirconia layers, the sintered body becomes a layered body on which subtle variations in texture are visible. When a texture more closely resembling natural teeth is sought, the sintered body according to this embodiment can be in a structure in which, for example, three or more and ten or fewer, three or more and six or fewer, four or more and seven or fewer or even four or more and six or fewer unit zirconia layers are stacked together.

The composition-gradient zirconia layer may have a unit zirconia layer other than the first and second composition-gradient zirconia layers (Hereinafter also referred to as "a composition-gradient intermediate zirconia layer.") between the first and second composition-gradient zirconia layers, and may include multiple unit zirconia layers as a composition-gradient intermediate zirconia layer. When a composition-gradient intermediate zirconia layer is included (In a composition-gradient intermediate zirconia layer, the first unit zirconia layer, lying adjacent to the first composition-gradient zirconia layer, is also referred to as "the third composition-gradient zirconia layer," and the second and subsequent unit zirconia layers, stacked from the third composition-gradient zirconia layer toward the back side, are also referred to as "the fourth composition-gradient zirconia layer," "the fifth composition-gradient zirconia layer," etc.), the composition-gradient intermediate zirconia layer has a structure in which the unit zirconia layers are stacked in such a manner that the amount of the stabilizing element remains unchanged or decreases in the direction opposite the stacking direction. That is, the amount of the stabilizing element remains unchanged or decreases starting from the third composition-gradient zirconia layer, followed by the fourth and then the fifth. If the variations in color based on synergistic effects, for example of the differences in the amount of the stabilizing element and translucency, are to be more visually perceptible, it is preferred that the composition-gradient intermediate zirconia layer have a structure in which the unit zirconia layers are stacked in such a manner that the amount of the stabilizing element decreases in the direction opposite the stacking direction.

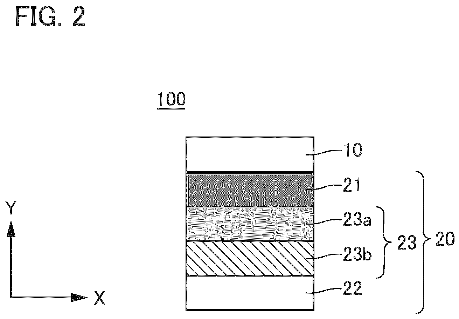

A sintered body according to this embodiment having a composition-gradient intermediate zirconia layer will now be described using Fig. 2.

Fig. 2 is a schematic view illustrating an example of a structure of a sintered body according to this embodiment, schematically illustrating a cross-section of a sintered body (100) having a surface zirconia layer (10) and a composition-gradient zirconia layer (20) composed of four unit zirconia layers. The sintered body illustrated in Fig. 2 has a structure in which a third composition-gradient zirconia layer (23a) and a fourth composition-gradient zirconia layer (23b), which constitute a composition-gradient intermediate zirconia layer (23), are stacked together in addition to a surface zirconia layer, a first composition-gradient zirconia layer (21) and a second composition-gradient zirconia layer (22) as described earlier herein. For the sintered body (100) according to this embodiment, the amount of the stabilizing element in the zirconia remains unchanged or decreases starting from the first composition-gradient zirconia layer (21), then the third composition-gradient zirconia layer (23a), followed by the fourth composition-gradient zirconia layer (23b) and then the second composition-gradient zirconia layer (22), and the amount of the stabilizing element in the zirconia containing at least one stabilizing element contained in the surface zirconia layer (10) is smaller than the amount of the stabilizing element in the zirconia containing at least one stabilizing element contained in the first composition-gradient zirconia layer (21).

In the following, the composition of the surface zirconia layer and the composition-gradient zirconia layer will be described.

<Composition of the Zirconia Layers>

As stated above, the surface zirconia layer and the unit zirconia layers are layers whose base constituent is zirconia (hereinafter also referred to as "zirconia layers"), and the zirconia is zirconia containing at least one stabilizing element (Hereinafter also referred to as "stabilizing element-containing zirconia."). The sintered body according to this embodiment and its zirconia layers may contain not only the stabilizing element-containing zirconia but also inevitable impurities, such as hafnia ($HfO_2$). The amount of hafnia as an inevitable impurity greatly varies depending on the source ore and the manufacturing method, but for example, it can be 2.0% by mass or less. In this embodiment, calculations of values relating to composition, such as the calculations of amounts and density, can be performed by calculating them assuming that hafnia is zirconia ($ZrO_2$).

For the sintered body according to this embodiment, the zirconia is preferably zirconia in a state in which zirconia resulting from the heat treatment of zirconia sol has been sintered, more preferably zirconia in a state in which zirconia resulting from the heat treatment of zirconia sol obtained via the hydrolysis of a zirconium compound has been sintered, even more preferably zirconia in a state in which zirconia resulting from the heat treatment of zirconia sol produced through the hydrolysis of zirconium oxychloride.

The zirconia contained in the zirconia layers can be, for example, sintered zirconia, i.e., zirconia crystal particles.

<<Stabilizing Element>>

The stabilizing element can be any element(s) having the function of reducing the phase transition of zirconia. The stabilizing element can be, for example, at least one stabilizing element that includes no element having the function of coloring zirconia but has the function of reducing phase transition (Hereinafter also referred to as "non-coloring stabilizing element."), at least one stabilizing element that includes an element having the function of coloring zirconia and has the function of reducing phase transition (Hereinafter also referred to as "coloring stabilizing element.") or both and may be at least one non-coloring stabilizing element and at least one coloring stabilizing element. Preferably, the stabilizing element includes at least a non-coloring stabilizing element, preferably is at least one non-coloring stabilizing element.

A specific example for the stabilizing element is one or more selected from the group of yttrium (Y), calcium (Ca), magnesium (Mg), cerium (Ce), praseodymium (Pr), neodymium (Nd), terbium (Tb), erbium (Er) and ytterbium (Yb) .

An example for the non-coloring stabilizing is one or more selected from the group of yttrium, calcium, magnesium and cerium. Preferably, the non-coloring stabilizing element is at least one of yttrium or cerium, more preferably yttrium.

An example for the coloring stabilizing element is one or more selected from the group of praseodymium, neodymium, terbium, erbium and ytterbium. Preferably, the coloring stabilizing element is at least one of terbium or erbium, more preferably erbium.

The stabilizing element is in a state in which it is contained in the zirconia and is in a state in which it is dissolved in the zirconia. The sintered body according to this embodiment preferably contains no undissolved stabilizing element, or, in other words, contains no stabilizing element not dissolved in zirconia. In this embodiment, "contains no undissolved stabilizing element" means that no XRD peak derived from a stabilizing element is observed in the XRD measurement and XRD pattern analysis described later herein. The inclusion of an undissolved stabilizing element, however, can be acceptable if the extent is such that no XRD peak derived from a stabilizing element is observed.

The amount of the stabilizing element in the zirconia contained in the surface zirconia layer (Hereinafter also referred to as "the stabilizing element content of the surface zirconia layer.") is preferably 2.5 mol% or more, more preferably 3.0 mol% or more, even more preferably 3.5 mol% or more, still more preferably 4 mol% or more, still more preferably 4.3 mol% or more, still more preferably 4.9 mol% or more. The stabilizing element content of the surface zirconia layer can be any percentage that is 6.0 mol% or less, 5.8 mol% or less, 5.5 mol% or less or 5.2 mol% or less. An example of a stabilizing element content of the surface zirconia layer can be 2.5 mol% or more and 6.0 mol% or less, 3.5 mol% or more and 5.2 mol% or less, 3.5 mol% or more and 6.0 mol% or less or 3.5 mol% or more and less than 5.2 mol%.

The amount of the stabilizing element in the zirconia contained in the unit zirconia layers (Hereinafter also referred to as "the stabilizing element content of the unit zirconia layers.") is preferably 2.5 mol% or more, more preferably 3.5 mol% or more, even more preferably 3.7 mol% or more, still more preferably 4.0 mol% or more, still more preferably 4.5 mol% or more, still more preferably 4.9 mol% or more. The stabilizing element content of the unit zirconia layers can be any percentage that is 6.0 mol% or less, 5.8 mol% or less, 5.5 mol% or less or 5.2 mol% or less. An example of a stabilizing element content of the unit zirconia layers can be 2.5 mol% or more and 6.0 mol% or less, 3.5 mol% or more and 5.5 mol% or less, 3.5 mol% or more and 6.0 mol% or less or 3.5 mol% or more and less than 5.5 mol%.

The stabilizing element content of the surface zirconia layer is smaller than the amount of the stabilizing element in the zirconia contained in the first composition-gradient zirconia layer (Hereinafter also referred to as "the stabilizing element content of the first composition-gradient zirconia layer."). Specifically, the stabilizing element content of the surface zirconia layer is smaller than the stabilizing element content of the first composition-gradient zirconia layer with the difference between the stabilizing element content of the surface zirconia layer and the stabilizing element content of the first composition-gradient zirconia layer being more than 0 mol%, preferably 0.05 mol% or more, more preferably 0.1 mol% or more, even more preferably 0.15 mol% or more, still more preferably 0.2 mol% or more. The difference, furthermore, can be any value that is 2.5 mol% or less, 1.5 mol% or less 1.0 mol% or less, 0.5 mol% or less or 0.4 mol% or less. When the difference falls within these ranges, it is likely that a sintered body that has high mechanical strength in its surface zirconia layer and with which the occurrence of chipping can be reduced is obtained. The difference between the stabilizing element content of the surface zirconia layer and the stabilizing element content of the first composition-gradient zirconia layer is more than 0 mol% and preferably is 2.5 mol% or less, 0.05 mol% or more and 1.5 mol% or less, 0.1 mol% or more and 1.0 mol% or less, 0.15 mol% or more and 0.5 mol% or less or 0.2 mol% or more and 0.4 mol% or less.

The stabilizing element content of the first composition-gradient zirconia layer can be any percentage that falls within the ranges specified above for the stabilizing element content of the unit zirconia layers and is greater than the amount in the surface zirconia layer.

The amount of the stabilizing element in the zirconia contained in the second composition-gradient zirconia layer (Hereinafter also referred to as "the stabilizing element content of the second composition-gradient zirconia layer.") can be any percentage that falls within the ranges specified above for the stabilizing element content of the unit zirconia layers. Preferably, it is greater than the stabilizing element content of the first composition-gradient zirconia layer.

The amount of the stabilizing element in the zirconia contained in each layer in the composition-gradient intermediate zirconia layer (Hereinafter also referred to as "the stabilizing element content of the composition-gradient intermediate zirconia layer.") is equal to or greater than the minimum and equal to or smaller than the maximum of the amounts of the stabilizing element in the zirconia contained in the first and second composition-gradient zirconia layers. The stabilizing element content of the composition-gradient intermediate zirconia layer, furthermore, remains unchanged or decreases from the surface zirconia layer in the direction opposite the stacking direction. If the variations in color based on synergistic effects, for example of the differences in the amount of the stabilizing element and translucency, are to be more visually perceptible, it is preferred that the stabilizing element content of the composition-gradient intermediate zirconia layer decrease from the surface zirconia layer toward the surface of the layered body opposite the surface zirconia layer.

The sintered body according to this embodiment preferably includes a structure in which the difference in the amount of the stabilizing element between adjacently stacked unit zirconia layers in the composition-gradient zirconia layer is 0.1 mol% or more, 0.3 mol% or more, 0.5 mol% or more or 1.0 mol% or more. The sintered body according to this embodiment, furthermore, preferably includes a structure in which the difference in the amount of the stabilizing element between adjacently stacked unit zirconia layers in the composition-gradient zirconia layer is 3.0 mol% or less, 2.5 mol% or less or 2.0 mol% or less.

The amount of the stabilizing element in the sintered body according to this embodiment (the amount of the stabilizing element in the sintered body as a whole) can be freely chosen, but is more than 1.5 mol%, preferably 2.5 mol% or more, more preferably 3.0 mol% or more, even more preferably 3.5 mol% or more, still more preferably 4.1 mol% or more, still more preferably 4.2 mol% or more. The amount of the stabilizing element in the sintered body according to this embodiment (the amount of the stabilizing element in the sintered body as a whole), furthermore, can be freely chosen, but preferably is less than 7.0 mol%, more preferably 6.5 mol% or less, even more preferably 6.0 mol% or less, still more preferably 5.5 mol% or less, still more preferably 5.3 mol% or less, still more preferably 5.0 mol% or less. These upper and lower limits may be in any combination. Consequently, the amount of the stabilizing element in the sintered body according to this embodiment (the amount of the stabilizing element in the sintered body as a whole) can be, for example, more than 1.5 mol% and less than 7.0 mol%, 2.5 mol% or more and 6.5 mol% or less, 3.0 mol% or more and 6.0 mol% or less or even 3.5 mol% or more and 5.8 mol% or less, preferably is 4.1 mol% or more and 5.5 mol% or less, more preferably 4.2 mol% or more and 5.3 mol% or less, even more preferably 4.2 mol% or more and 5.0 mol% or less. The stabilizing element content of the sintered body is determined from the equation below and varies depending on the thickness of each layer.

The stabilizing element content of the sintered body = (thickness of the surface zirconia layer/height of the sintered body) $\times$ the amount of the stabilizing element in the stabilizing element-containing zirconia contained in the surface zirconia layer + (thickness of each unit zirconia layer/height of the sintered body) $\times$ the amount of the stabilizing element in the stabilizing element-containing zirconia contained in each unit zirconia layer

In this embodiment, the amounts of the stabilizing element are molar percentages of the stabilizing element converted into an oxide to the total of zirconia and the stabilizing element converted into an oxide. The conversion of the stabilizing element into an oxide can be done by using $Y_2O_3$ for yttrium oxide, $CaO$ for calcium oxide, $MgO$ for magnesium oxide, $CeO_2$ for cerium oxide, $Pr_6O_{11}$ for praseodymium oxide, $Nd_2O_3$ for neodymium oxide, $Tb_4O_7$ for terbium oxide, $Er_2O_3$ for erbium oxide and $Yb_2O_3$ for ytterbium oxide. For example, when yttrium and erbium are contained as stabilizing elements, the amount of the stabilizing element (mol%) is determined from $(Y_2O_3 + Er_2O_3) / (ZrO_2 + Y_2O_3 + Er_2O_3) \times 100$.

<<Coloring Element>>

The coloring element in this embodiment is element(s) having the function of coloring zirconia. A specific example for the coloring element is at least one transition metal element, at least one lanthanoid-series rare earth element or both. Preferably, the coloring element is, for example, one or more selected from the group of iron (Fe), cobalt (Co), nickel (Ni), manganese (Mn), titanium (Ti), praseodymium (Pr), neodymium (Nd), europium (Eu), gadolinium (Gd), terbium (Tb), erbium (Er) and ytterbium (Yb), more preferably one or more selected from the group of iron, cobalt, manganese, titanium, praseodymium, gadolinium, terbium and erbium, even more preferably one or more selected from the group of iron, cobalt, titanium and erbium.

The coloring element contained in the sintered body according to this embodiment only needs to be at least one of coloring elements contained dissolved in the zirconia, such as coloring elements contained in the state of an oxide (Hereinafter also referred to as "oxide coloring elements.") and coloring stabilizing elements. An example of preferred oxide coloring element(s) is one or more selected from the group of iron, cobalt, nickel, titanium and manganese, and it is more preferred that the oxide coloring element(s) be one or more selected from the group of iron, cobalt and titanium. The sintered body according to this embodiment may contain two or more coloring elements; for example, it may contain two or more and five or fewer or even three or more and four or fewer coloring elements. The sintered body according to this embodiment preferably contains at least a coloring stabilizing element, more preferably at least one of terbium or erbium, even more preferably at least erbium. Examples of particularly preferred coloring elements include one or more selected from the group of iron, titanium and erbium; cobalt, titanium and erbium; and iron and erbium, cobalt, titanium and erbium, iron and erbium or both and iron and erbium.

The type and amount of the coloring element contained in the surface zirconia layer and the unit zirconia layers are not particularly restricted; it would be beneficial for them to be selected as appropriate considering, for example, color gradations between the layers.

For the amount of the coloring element in the sintered body according to this embodiment, the lower limit is more than 0% by mass, preferably 0.01% by mass or more, more preferably 0.03% by mass or more, even more preferably 0.04% by mass or more as a percentage by mass of the coloring element converted into an oxide in relation to the mass of the sintered body according to this embodiment. The upper limit, furthermore, is 1.5% by mass or less, preferably 1.0% by mass or less, more preferably 0.8% by mass or less, even more preferably 0.3% by mass or less. These upper and lower limits may be in any combination. Consequently, the amount of the coloring element in the sintered body according to this embodiment can be any value that is, for example, more than 0% by mass and 1.5% by mass or less, preferably 0.01% by mass or more and 1.0% by mass or less, more preferably 0.03% by mass or more and 0.8% by mass or less, even more preferably 0.04% by mass or more and 0.3% by mass or less as a percentage by mass of the coloring element converted into an oxide in relation to the mass of the sintered body according to this embodiment. For example, when yttrium (a non-coloring stabilizing element) and erbium (a coloring stabilizing element) are contained as stabilizing elements with alumina and cobalt (an oxide coloring element) also contained, the amount of the coloring element (% by mass) is determined from $(Co_3O_4 + Er_2O_3)/(ZrO_2 + Y_2O_3 + Er_2O_3 + Co_3O_4 + Al_2O_3) \times 100$. The conversion of the coloring element into an oxide can be done by using $Pr_6O_{11}$ for praseodymium oxide, $Nd_2O_3$ for neodymium oxide, $Tb_4O_7$ for terbium oxide, $Er_2O_3$ for erbium oxide, $Yb_2O_3$ for ytterbium oxide, $Fe_2O_3$ for iron oxide, $Co_3O_4$ for cobalt oxide, $NiO$ for nickel oxide, $Mn_3O_4$ for manganese oxide and $TiO_2$ for titanium oxide.

The surface zirconia layer may contain at least one coloring element. For the amount of the coloring element contained in the surface zirconia layer, the lower limit is more than 0% by mass, preferably 0.01% by mass or more, more preferably 0.03% by mass or more, even more preferably 0.04% by mass or more as a percentage by mass of the coloring element converted into an oxide in relation to the mass of the sintered body according to this embodiment. The upper limit can be any value that is 2% by mass or less, preferably 1.2% by mass or less, more preferably 0.8% by mass or less, even more preferably 0.3% by mass or less. These upper and lower limits may be in any combination. Consequently, the amount of the coloring element contained in the surface zirconia layer can be, for example, more than 0% by mass and 2% by mass or less, preferably 0.01% by mass or more and 1.2% by mass or less, more preferably 0.03% by mass or more and 0.8% by mass or less, even more preferably 0.04% by mass or more and 0.3% by mass or less as a percentage by mass of the coloring element converted into an oxide in relation to the mass of the sintered body according to this embodiment.

For the amount of the coloring element contained in the unit zirconia layers, it is preferred that the lower limit be more than 0% by mass, preferably 0.01% by mass or more, more preferably 0.03% by mass or more, even more preferably 0.04% by mass or more as a percentage by mass of the coloring element converted into an oxide in relation to the mass of the sintered body according to this embodiment, and that the upper limit be 2% by mass or less, preferably 1.2% by mass or less, more preferably 0.8% by mass or less, even more preferably 0.3% by mass or less. These upper and lower limits may be in any combination. Consequently, the amount of the coloring element contained in the unit zirconia layers is preferably, for example, more than 0% by mass and 2% by mass or less, preferably 0.01% by mass or more and 1.2% by mass or less, more preferably 0.03% by mass or more and 0.8% by mass or less, even more preferably 0.04% by mass or more and 0.3% by mass or less as a percentage by mass of the coloring element converted into an oxide in relation to the mass of the sintered body according to this embodiment.

For the sintered body according to this embodiment, it is preferred that the difference in the amount of the coloring element between adjacently stacked zirconia layers be 0% by mass or more, preferably 0.002% by mass or more, more preferably 0.005% by mass or more, even more preferably 0.01% by mass or more, even more preferably 0.015% by mass or more, still more preferably 0.02% by mass or more. With increasing differences in coloring element content, differences in color between zirconia layers tend to increase, but simultaneously, warpage can also become greater. When the difference in the amount of the coloring element between adjacent unit layers is less than 1% by mass, 0.8% by mass or less or even 0.5% by mass or less, the variations in the color of the sintered body are likely to be variations in color comparable to those of natural teeth. The difference in the amount of the coloring element between adjacent unit layers is preferably 0% by mass or more and less than 1% by mass, 0.002% by mass or more and 0.8% by mass or less or 0.005% by mass or more and 0.5% by mass or less, and it is preferred that the differences in the amount of the coloring element in all pairs of adjacent unit layers satisfy this.

The sintered body according to this embodiment may contain alumina. It is preferred that at least one zirconia layer contain alumina, and it is more preferred that the surface zirconia layer and the composition-gradient zirconia layer contain alumina. When the sintered body according to this embodiment contains alumina, the alumina content can be any percentage that is 0% by mass or more as a percentage of the mass of the alumina in relation to the mass of the sintered body; for example, it can be 0% by mass or more and 0.15% by mass or less, 0% by mass or more and 0.10% by mass or less or even 0% by mass or more and 0.07% by mass or less. When alumina is contained, the lower limit to the alumina content, for example, is more than 0% by mass, preferably 0.005% by mass or more, more preferably 0.01% by mass or more, and the upper limit to the alumina content can be, for example, 0.15% by mass or less, preferably 0.10% by mass or less, more preferably 0.07% by mass or less. These upper and lower limits may be in any combination. Consequently, the alumina content can be, for example, more than 0% by mass and 0.15% by mass or less, preferably 0.005% by mass or more and 0.10% by mass or less, more preferably 0.01% by mass or more and 0.07% by mass or less.

The alumina content of the zirconia layers, too, can be any percentage that falls within the same ranges as specified above. The alumina content of the zirconia layers can affect the thermal contraction behavior during the calcination stage. The alumina content of the zirconia layers can be freely chosen, and each of the zirconia layers may vary in alumina content; however, it is preferred that the alumina content be identical therebetween. When the alumina content is different between the zirconia layers, the lower limit to the difference in alumina content between adjacent zirconia layers, for example, can be more than 0% by mass or even 0.005% by mass or more, and the upper limit to the difference in alumina content between adjacent zirconia layers can be, for example, 0.15% by mass or less, 0.1% by mass or less or even 0.01% by mass or less. These upper and lower limits may be in any combination. Consequently, the difference in alumina content between adjacent zirconia layers can be, for example, more than 0% by mass and 0.15% by mass or less, more than 0% by mass and 0.1% by mass or less, more than 0% by mass and 0.01% by mass or less or even 0.05% by mass or more and 0.01% by mass or less. For example, in the case of a zirconia layer containing alumina ($Al_2O_3$) and made of zirconia in which the non-coloring stabilizing element is yttrium and in which the coloring stabilizing element is erbium, the alumina content can be determined as $Al_2O_3/ (ZrCt + Y_2O_3 + Er_2O_3 + Al_2O_3)\} \times 100$ (% by mass).

The sintered body according to this embodiment is preferably substantially free of silica ($SiO_2$). More preferably, the amount of silica is at or below the detection limit.

The sintered body according to this embodiment preferably includes a zirconia layer containing zirconia whose crystal phase(s) is at least one of the tetragonal phase (T phase) or the cubic phase (C phase), more preferably includes at least a zirconia layer containing zirconia in which the tetragonal phase is its primary phase, even more preferably includes a zirconia layer containing zirconia in which the tetragonal phase is its primary phase and a zirconia layer containing zirconia in which the cubic phase is its primary phase. It should be noted that "the primary phase" in this embodiment refers to the crystal phase in zirconia whose relative abundance (proportion of the integrated intensity of the peak) is the highest among them. This relative abundance can be determined from an XRD pattern of the sintered body's surface.

An example of conditions for the measurement of an XRD pattern of the sintered body's surface is the following conditions.

X-ray source: CuK$\alpha$ radiation ($\lambda$ = 1.541862 Å)
Measurement mode: Continuous scanning
Scan speed: 4°/minute

Measurement range: 2θ = 26° to 33°
Acceleration voltage and current: 40 mA and 40 kV
Vertical Seller slits: 10 mm
Divergence/Incident slits: 1°
Receiving slits: Open
Detector: Semiconductor detector (D/teX Ultra)
Filter: Ni filter
Goniometer radius: 185 mm

The resulting XRD pattern is subjected to smoothing treatment and background subtraction treatment, and profile fitting is performed using the split pseudo-Voigt function to determine the percentages of the tetragonal and cubic phases (percentages of the integrated intensities of the peaks). The crystal phase with the higher percentage can be designated as the primary phase. The measurement and fitting of the XRD pattern can be conducted using a common powder x-ray diffractometer (e.g., Ultima IV, manufactured by RIGAKU CORPORATION) and an analysis program that comes with the x-ray diffractometer (e.g., PDXL integrated powder x-ray analysis software Ver. 2.2, manufactured by RIGAKU CORPORATION).

<Characteristics of the Sintered Body and Each Zirconia Layer (each sintered body layer) Constituting the Sintered Body>

The sintered bodies (100) illustrated in Figs. 1 and 2 are depicted with the thickness of each layer being similar. For the sintered body according to this embodiment, however, the thickness of each layer (Hereinafter also referred to as "the layer thickness.") may vary from layer to layer, and the thickness of any of the layers may be large. If the texture is to more closely resemble that of natural teeth, it is preferred that the thickness of the surface zirconia layer be smaller than the thickness of the composition-gradient zirconia layer, and, furthermore, it is preferred that the thickness of the surface zirconia layer be small compared with the thickness of the unit zirconia layers constituting the composition-gradient zirconia layer.

The layer thickness ratio between the surface zirconia layer and the composition-gradient zirconia layer is not particularly restricted, but preferably is from 1:1 to 1:50, more preferably from 1:1 to 1:30, even more preferably from 1:2 to 1:20 as the thickness of the surface zirconia layer:the thickness of the composition-gradient zirconia layer.

The shape of the sintered body according to this embodiment can be freely chosen; the sintered body only needs to be in at least one shape selected from the group of spherical, ellipsoidal, disk-shaped, columnar, cubic, cuboid and polygonal, a shape suitable for use as a dental material, typically a crown, bridge, inlay, onlay or other dental prosthetic materials, or any other shape selected according to the intended purpose of use. It should be noted that in this embodiment, spherical shapes include objects with ball shapes that are not perfectly spherical, such as substantially spherical shapes, and polygonal shapes may include shapes like polygons, such as substantially polygonal, besides polygons.

The dimensions of the sintered body according to this embodiment can be freely chosen; for example, they can be longitudinally 10 mm or more and 120 mm or less, transversely 12 mm or more and 120 mm or less and in height 6 mm or more and 40 mm or less. Moreover, the thickness of the sintered body according to this embodiment in the stacking direction, or the height of the sintered body, can be freely chosen, but for example, it can be 4 mm or more and 40 mm or less or even 5 mm or more and 30 mm or less.

<<Warpage and the Amount of Deformation of the Sintered Body>>

Warpage in this embodiment is a value measured using thickness gauges conforming to JIS B 7524: 2008 (Hereinafter also referred to simply as "gauges."). The warpage of the sintered body according to this embodiment is preferably 1.0 mm or less, more preferably 0.3 mm or less, even more preferably 0.2 mm or less, still more preferably 0.1 mm or less, particularly preferably 0.05 mm or less. Although it is preferred that the sintered body have no warpage (the warpage be 0 mm), the sintered body according to this embodiment may have an extent of warpage that cannot be measured with gauges (the warpage may be 0 mm or more). The sintered body according to this embodiment can have a warpage of, for example, greater than 0 mm or even 0.01 mm or more. The warpage is preferably 0.06 mm or less, 0.05 mm or less or even below the measurement limit (less than 0.03 mm). The warpage of the sintered body according to this embodiment can be, for example, below the measurement limit, 0 mm or more and 0.06 mm or less, 0 mm or more and 0.05 mm or less or 0 mm or more and less than 0.03 mm.

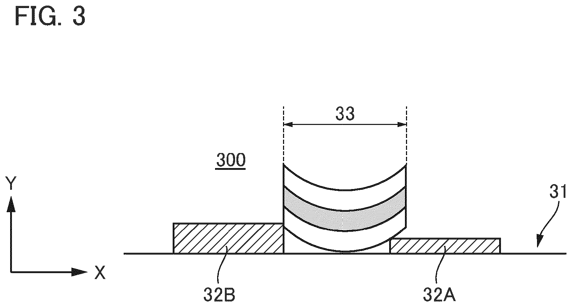

The warpage can be measured as the maximum thickness of gauges that can be inserted into a gap that is created in a state in which the sintered body (layered body) is positioned in such a manner that its outwardly curved portion is in contact with a horizontal plate. Fig. 3 is a schematic view illustrating a method for measuring the warpage. The sintered body (300) presents a cross-section of a disk-shaped sample, illustrating a sintered body in a state in which it is warped in the stacking direction (Y-axis direction). In Fig. 3, the sintered body (300) is illustrated with emphasis on its warpage for explanatory purposes. As illustrated in Fig. 3, in the measurement of the warpage, the sintered body (300) in an uneven shape is positioned in such a manner that its outwardly curved portion is in contact with a horizontal plate (31). As a result of this, a gap is created between the horizontal plate (31) and the surface at which the sintered body (300) and the horizontal plate (31) are in contact with each other (Hereinafter also referred to as "the bottom surface."). Gauges are inserted into this gap, and the warpage can be measured as the maximum thickness of gauges that can be inserted. In Fig. 3, the gauge (32A) is positioned below the bottom surface of the sintered body (300), illustrating a state in which it has been successfully inserted into the gap. The gauge (32B), by contrast, is not positioned below the bottom surface of the sintered body (300), illustrating a state in which it cannot be inserted into the gap. The gauges (32A) and (32B) in Fig. 3 are gauges with a one-step difference (e.g., 0.01 mm) in thickness therebetween, and the warpage of the sintered body (300) is the thickness of the gauge (32A). It should be noted that in Fig. 3, the drawing is presented as a diagram in which both of the gauges (32A) and (32B) have been inserted for the sake of brevity in explanation. In the measurement of the warpage, however, the warpage can be measured by inserting the thinnest gauge into the gap first, followed by thicker gauges in sequence (e.g., the warpage is measured using the gauge (32A) first, then it is removed, and then the warpage is measured using the thicker gauge (32B)).

For the sintered body according to this embodiment, it is preferred that the warpage in relation to a dimension of the sintered body (Hereinafter also referred to as "the amount of deformation.") be 1.0 or less, more preferably 0.5 or less, more preferably 0.2 or less, even more preferably 0.15 or less. The amount of deformation can be, for example, 0 or greater, 0.01 or greater or even 0.05 or greater.

The amount of deformation can be determined from the following equation.

The amount of deformation = (warpage: mm)/(dimension of the sintered body: mm) $\times$ 100

The dimension of the sintered body is the size of the sintered body in the direction perpendicular to the direction of the warpage. The sintered body (300) in Fig. 3 is warped in the stacking direction (Y-axis direction), which means that the dimension of the sintered body (300) is the sintered body's size (33) in the horizontal direction (X-axis direction), which is perpendicular to the stacking direction. The dimension can be measured by a known measuring method in which a caliper or micrometer, for example, is used. For instance, when the sintered body is a disk-shaped or columnar layered body, the dimension of the sintered body refers to the diameter of the sintered body. When the sintered body is a disk-shaped or columnar layered body, the dimension can be obtained by, for example, measuring the diameter at the upper end and the diameter at the lower end at four points each using a caliper, determining the average diameters at the upper and lower ends and using the average of the determined values as the dimension of the layered body.

In this embodiment, warpage and the amount of deformation are preferably values measured using a sample in a disk shape as the sample for measurement, more preferably values measured using, as the sample for measurement, a sample in a disk shape having a diameter of 5 mm or more and 120 mm or less.

For example, when the sintered body has a diameter of 105 mm and a warpage of 0.2 mm, the amount of deformation is 0.2/105 $\times$ 100 = 0.19.

<<Density of the Sintered Body>>

For the sintered body according to this embodiment, the lower limit to its density as measured by a method according to JIS R 1634 can be, for example, 5.7 g/cm$^3$ or more, 5.9 g/cm$^3$ or more or 6.0 g/cm$^3$ or more, and the upper limit to its density can be 6.3 g/cm$^3$ or less or 6.1 g/cm$^3$ or less. These upper and lower limits may be in any combination. Consequently, the density of the calcined body can be, for example, 5.7 g/cm$^3$ or more and 6.3 g/cm$^3$ or less, 5.9 g/cm$^3$ or more and 6.1 g/cm$^3$ or less or 6.0 g/cm$^3$ or more and 6.1 g/cm$^3$ or less. Densities within these ranges correspond to 99% or more as relative densities and are densities that make the sintered body one having practical strength, or a so-called dense one.

<<Color of the Sintered Body or Sintered Body Layers>>

For the color of the sintered body according to this embodiment or each of its zirconia layers (each sintered body layer), the lower limit to lightness L*, indicated in the L*a*b* color system, can be, for example, 60 or greater, preferably 65 or greater, and the upper limit to lightness L* can be 90 or less, preferably 85 or less. The lower limit to hue a* can be -5 or greater, preferably -3 or greater, and the upper limit to hue a* can be 5 or less, preferably 3 or less. The lower limit to hue b* can be 0 or greater, preferably 3 or greater, and the upper limit to hue b* can be 25 or less, preferably 20 or less. These upper and lower limits may be in any combination. Consequently, the color of the sintered body according to this embodiment or each of its zirconia layers (each sintered body layer) can be, for example, The lightness L* can be 60 or greater and 90 or less, preferably 65 or greater and 85 or less. The hue a* can be -5 or greater and 5 or less, preferably -3 or greater and 3 or less, and the hue b* can be 0 or greater and 25 or less, preferably 3 or greater and 20 or less. When the color falls within these ranges, the sintered body exhibits a color resembling that of natural teeth.

The sintered body according to this embodiment preferably has varying chromas C* between its zirconia layers to have variations in color. Chroma C* is a measure of vividness and can be determined as $C* = \{(a*)^2 + (b*)^2\}^{0.5}$ from hues a* and b* indicated in the L*a*b* color system. The lower limit to the absolute difference in chroma C* ($\Delta$C*) between adjacently stacked unit zirconia layers in the composition-gradient zirconia layer can be, for example, 0.1 or greater, 0.3 or greater, 1.0 or greater or even 1.5 or greater, and the upper limit to the absolute difference ($\Delta$C*) can be 15.0 or less, 10.0 or less, 5.0 or less or even 3.0 or less. These upper and lower limits may be in any combination. Consequently, for the sintered body according to this embodiment, the absolute difference in chroma C* ($\Delta$C*) between adjacently stacked unit zirconia layers can be, for example, 0.1 or greater and 15.0 or less, 0.3 or greater and 10.0 or less, 1.0 or greater and 5.0 or less or even 1.5 or greater and 3.0 or less. When $\Delta$C* falls within these ranges, the sintered body can be visually perceived as a layered body having gradations in vividness closely resembling natural teeth. Likewise, the absolute difference in lightness L* ($\Delta$L*) between adjacently stacked unit zirconia layers is preferably, for example, 0.1 or greater, more preferably 0.5 or greater, and preferably is 15.0 or less, more preferably 10.0 or less.

The color (L*, a* and b*) and C* can be determined using a spectrocolorimeter equipped with illuminating and receiving optical systems conforming to geometric conditions c in JIS Z 8722 and values measured by the SCI method. An example of a spectrocolorimeter is CM-700d, manufactured by Konica Minolta, Inc. The measurement of the color of the sintered body according to this embodiment or the sintered body layers can be performed using a sample for measurement obtained by cutting out any point of the sintered body in the horizontal direction and working the cutout into a disk shape to achieve a diameter of 20 mm $\times$ a thickness of 1.0 $\pm$ 0.1 mm and a surface roughness (Ra) $\leq$ 0.02 um.

An example of specific conditions for the measurement of the color and C* is the following conditions in the method of measuring them with the sample for measurement placed on a black board (so-called black background measurement).

Light source: D65 light source
Viewing angle: 10°
Measurement method: SCI

<<Transmittance of the Sintered Body or Sintered Body Layers>>

The sintered body according to this embodiment preferably includes at least a zirconia layer having a translucent appearance. It is, furthermore, preferred that the sintered body have at least a zirconia layer whose total transmittance for CIE standard illuminant D65 at a sample thickness of 1.0 $\pm$ 0.1 mm (Hereinafter also referred to simply as "total transmittance.") is preferably 15% or more, more preferably 20% or more, even more preferably 23% or more. Moreover, it is preferred that the sintered body have a zirconia layer whose total transmittance is preferably 60% or less, more preferably 55% or less, even more preferably 50% or less. The sintered body according to this embodiment absorbs different wavelengths of light depending on its exhibited color. For use as the measure of translucency, therefore, the total transmittance for light including different wavelengths, like CIE standard illuminant D65, is suitable.

For each zirconia layer in the sintered body according to this embodiment (each sintered body layer), it is preferred that the difference in total transmittance between adjacently stacked zirconia layers be 1% or more, more preferably 1.5% or more. It is, furthermore, preferred that the difference in total transmittance between adjacently stacked zirconia layers be 10% or less, more preferably 5% or less.

The total transmittance of the sintered body according to this embodiment can be measured as a measurement sample obtained by cutting out any point of the sintered body in the horizontal direction and working the cutout to achieve a sample thickness of 1.0 $\pm$ 0.1 mm.

The total transmittance can be measured by a method according to JIS K 7361 and determined as a transmittance value totaling diffuse transmittance and linear transmittance for incident light, using CIE standard illuminant D65 as the incident light. With a sample measuring 1.0 $\pm$ 0.1 mm in thickness with a surface roughness (Ra) $\leq$ 0.02 um as a sample for measurement, the sample is illuminated with light from CIE standard illuminant D65 using a common haze meter (e.g., NDH4000, manufactured by NIPPON DENSHOKU). The transmittance (diffuse and linear transmittance) of the sample is measured by collecting transmitted light using an integrating sphere, and it can be used as the total transmittance.

<<Three-Point Flexural Strength of the Sintered Body Layers>>

For the three-point flexural strength of each zirconia layer in the sintered body according to this embodiment (each sintered body layer), it is preferred that the three-point flexural strength as measured by a method according to JIS R 1601 be 500 MPa or more, more preferably 550 MPa or more, more preferably 600 MPa or more, even more preferably 800 MPa or more. The three-point flexural strength can be, for example, less than 1300 MPa or even 1200 MPa or less, preferably is 500 MPa or more and less than 1300 MPa or 800 MPa or more and 1200 MPa or less.

The three-point flexural strength of the surface zirconia layer (sintered body layer) as measured by a method according to JIS R 1601 is preferably 700 MPa or more, more preferably 750 MPa or more, even more preferably 800 MPa or more. When the three-point flexural strength of the surface zirconia layer (sintered body layer) is 700 MPa or more, the occurrence of chipping can be reduced sufficiently.

For the reduction of the occurrence of chipping, the surface zirconia layer preferably has a three-point flexural strength higher than that of the first composition-gradient zirconia layer, with the difference therebetween being 50 MPa or more, 100 MPa or more or 150 MPa or more. The difference in three-point flexural strength between the surface zirconia layer and the first composition-gradient zirconia layer can be freely chosen unless aesthetics is impaired; however, it can be any difference that is 500 MPa or less or even 300 MPa or less.

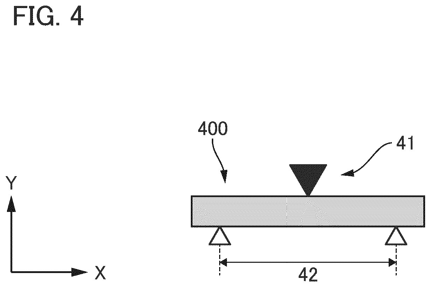

Fig. 4 is a schematic view illustrating how the three-point flexural strength of a zirconia layer (sintered body layer) (400) is measured. In Fig. 4, the stacking direction is presented in the Y-axis direction, and the horizontal direction is presented in the X-axis direction. The sample for measurement subjected to the measurement of three-point flexural strength is a sintered body in a cuboid shape prepared with its thickness as the stacking direction and its width and length as the horizontal direction. As illustrated in Fig. 4, the three-point flexural strength can be measured by applying a load (41) in such a manner that it will be perpendicular to the length of the sample for measurement (400). The sample for measurement can be positioned in a manner as to allow the load (41) to be applied to the middle of the span (42). In the measurement of the three-point flexural strength, a sintered body in a prismatic shape measuring 4 mm in width and 3 mm in thickness with a span of 30 mm is used as the sample for measurement, and an average of ten measurements taken with the crosshead speed set to 0.5 mm/min can be used as the three-point flexural strength of the zirconia layer (sintered body layer) according to this embodiment.

In the following, while an embodiment in which the layered body is a calcined body is presented, major differences from the sintered body described above will be described.

(Calcined Body)

A calcined body according to this embodiment is a calcined body including:

a surface zirconia composition layer containing zirconia that contains at least one stabilizing element and also has a necking structure and a composition-gradient zirconia composition layer composed of two or more unit zirconia composition layers,

the unit zirconia composition layers each containing at least one coloring element and zirconia that contains at least one stabilizing element and also has a necking structure, and

the composition-gradient zirconia composition layer being constructed as a result of the unit zirconia composition layers being stacked in such a manner that the amount of the stabilizing element in the zirconia containing at least one stabilizing element contained in the composition-gradient zirconia composition layer remains unchanged or decreases from the surface zirconia composition layer toward the surface of the calcined body opposite the surface zirconia composition layer.

The amount of the stabilizing element in the zirconia containing at least one stabilizing element contained in the surface zirconia composition layer is smaller than the amount of the stabilizing element in the zirconia containing at least one stabilizing element contained in a first composition-gradient zirconia composition layer, which is one of the unit zirconia composition layers constituting the composition-gradient zirconia composition layer and is adjacent to the surface zirconia composition layer.

The calcined body is a composition having a multilayer structure, or is a so-called layered body, and is a layered body made up of tissues having a necking structure, or of so-called calcined particles. The calcined body is processed as needed, can be used as a precursor to a sintered body and is also referred to as a pre-sintered body, soft-sintered body or half-sintered body.

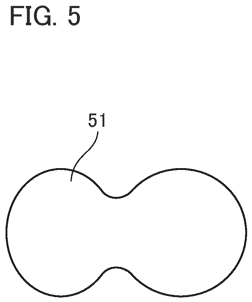

A necking structure is a structure that zirconia thermally treated below its sintering temperature has, and is a structure in which zirconia particles chemically adhere to one another. As illustrated in Fig. 5, in the zirconia (51) contained in the surface zirconia composition layer and unit zirconia composition layers (hereinafter also referred to as "zirconia composition layers") that the calcined body has, part of the shape of the particles of zirconia within the powder composition can be observed. In this embodiment, the tissue having a necking structure is a structure made of zirconia in its early stage of sintering. This is different from sintered tissue, or from a structure made up of zirconia crystal particles in their late stage of sintering. The calcined body according to this embodiment, therefore, can also be considered a layered body including three or more layers containing zirconia made up of zirconia particles having a necking structure of zirconia containing at least one stabilizing element and, if it contains at least one coloring element, containing the coloring element.

The calcined body has zirconia composition layers containing zirconia that contains at least one stabilizing element and also has a necking structure and, if it contains at least one coloring element, containing the coloring element (Hereinafter also referred to as "composition layers.") instead of having zirconia layers, and has a structure equivalent to the layered body structure described with the sintered body. In the calcined body, the stabilizing element and the coloring element may be in a state in which they are dissolved in the zirconia or may be in a state of oxides or their precursors.

<Composition of the Zirconia Composition Layers>

The zirconia contained in the calcined body is preferably in a state in which zirconia resulting from the heat treatment of zirconia sol has been thermally treated below its sintering temperature, more preferably in a state in which zirconia resulting from the heat treatment of zirconia sol obtained via the hydrolysis of a zirconium compound has been thermally treated below its sintering temperature, even more preferably in a state in which zirconia resulting from the heat treatment of zirconia sol produced through the hydrolysis of zirconium oxychloride has been thermally treated below its sintering temperature.

The amounts of the stabilizing element in the calcined body and each composition layer can be freely chosen; however, they can be any percentages similar to those in the sintered body according to this embodiment described above.

The calcined body more preferably includes at least a zirconia composition layer containing zirconia in which the tetragonal or cubic phase is its primary phase.

<Characteristics of the Calcined Body and the Layers Constituting the Calcined Body>

For the calcined body, it is preferred that the warpage be 1.0 mm or less, more preferably 0.5 mm or less, even more preferably 0.3 mm or less, still more preferably 0.2 mm or less, still more preferably 0.1 mm or less, still more preferably 0.05 mm or less. Although it is preferred that the calcined body have no warpage (the warpage be 0 mm), the calcined body may have an extent of warpage that cannot be measured with gauges (the warpage may be 0 mm or more). The calcined body can have a warpage of, for example, greater than 0 mm or even 0.01 mm or more. The warpage is preferably 0.06 mm or less, 0.05 mm or less or even below the measurement limit (less than 0.03 mm).

For the calcined body, it is preferred that the amount of deformation be 1.0 or less, more preferably 0.5 or less, even more preferably 0.2 or less, still more preferably 0.15 or less. The amount of deformation can be, for example, 0 or greater, 0.01 or greater or even 0.05 or greater.

For the calcined body, it is preferred that the density be 2.4 g/cm$^3$ or more, more preferably 3.1 g/cm$^3$ or more. The density is preferably 3.7 g/cm$^3$ or less, more preferably 3.5 g/cm$^3$ or less. Densities within these ranges correspond to 40% to 60% for relative densities. The calcined body can be a layered body having any level of strength suitable for working, such as CAD/CAM working.

The density of the calcined body can be determined from the mass, which can be determined by mass measurement, and the volume, which can be determined by dimensional measurement.

The color of the zirconia composition layers included in the calcined body may be different from that of a sintered body obtained by sintering the calcined body, or there may be no changes in color.

The calcined body and each zirconia composition layer are nontransparent and have a total transmittance of 0%. When measurement errors are considered, however, the total transmittance can be, for example, 0% or more and 0.2% or less.

The calcined body can have any level of strength such that defects during working, such as CAD/CAM or cutting, are unlikely to occur. For example, the strength can be 25 HV or more and 150 HV (= kgf/mm$^2$) or less or 30 HV or more and 130 HV or less as a Vickers hardness.

The measurement of the Vickers hardness can be performed using a common Vickers tester equipped with a diamond indenter with a square pyramid (e.g., Q30A, manufactured by Qness). In the measurement, the indenter is statically pressed onto the surface of the sample for measurement, and the diagonal length of the indentation created in the surface of the sample for measurement is measured visually. Using the obtained diagonal length, the Vickers hardness can be determined from the following equation.

$$Hv = F/\{d^2/2\sin(\alpha/2)\}$$

In this equation, Hv is the Vickers hardness (HV), F is the measurement load (1 kgf), d is the diagonal length (mm) of the indentation, and $\alpha$ is the angle between the opposite faces of the indenter (136°).

An example of conditions for the measurement of the Vickers hardness is the following conditions.

Sample for measurement: Disk-shaped sample with a thickness of 2.0 $\pm$ 0.5 mm
Measurement load: 1 kgf

Prior to the measurement, the sample for measurement is obtained by cutting out any point of the calcined body in the horizontal direction. Polishing the measurement surface with #800 waterproof abrasive paper to remove irregularities greater than 0.1 mm can be performed as a pretreatment.

The layered body according to this embodiment can be used for known applications of zirconia, such as decorative components, structural materials and optical materials. When the layered body is a sintered body, however, it is suitable for use as a dental material for dental restorations, such as crowns and bridges, and can be made into a dental material including the sintered body according to this embodiment. When the layered body is a calcined body, furthermore, it is suitable for use as a precursor to dental materials for dental restorations, such as crowns and bridges, and can be used as a prosthetic material for dentistry, such as a blank, disk, block or mill blank, and a precursor to them. The layered body, furthermore, can be made into a dental material including the layered body according to this embodiment.

(Method for Manufacturing the Layered body)

A method for manufacturing the layered body according to this embodiment will now be described.

A manufacturing method in the configuration in which the layered body according to this embodiment is a sintered body is:

a method for manufacturing a layered body, the method comprising a step of sintering a green body at 1200°C or above and 1600°C or below,
the green body having a surface powder composition layer containing zirconia containing at least one stabilizing element and a composition-gradient powder composition layer composed of two or more unit powder composition layers,
the unit powder composition layers each containing at least one coloring element and zirconia containing at least one stabilizing element,
the composition-gradient powder composition layer being constructed as a result of the unit powder composition layers being stacked in such a manner that the amount of the stabilizing element in the zirconia containing at least one stabilizing element contained in the composition-gradient powder composition layer remains unchanged or decreases from the surface powder composition layer toward the surface powder composition of the green body opposite the surface powder composition layer, and
the amount of the stabilizing element in the zirconia containing at least one stabilizing element contained in the surface powder composition layer being smaller than the amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

Another manufacturing method according to this embodiment, furthermore, is:

a method for manufacturing a layered body, the method comprising a step of calcining a green body at 800°C or above and below 1200°C to turn the green body into a calcined body and
a step of sintering the calcined body at 1200°C or above and 1600°C or below,
the green body having a surface powder composition layer containing zirconia containing at least one stabilizing element and a composition-gradient powder composition layer composed of two or more unit powder composition layers,
the unit powder composition layers each containing at least one coloring element and zirconia containing at least one stabilizing element,
the composition-gradient powder composition layer being constructed as a result of the unit powder composition layers being stacked in such a manner that the amount of the stabilizing element in the zirconia containing at least one stabilizing element contained in the composition-gradient powder composition layer remains unchanged or decreases from the surface powder composition layer toward the surface powder composition of the green body opposite the surface powder composition layer, and
the amount of the stabilizing element in the zirconia containing at least one stabilizing element contained in the surface powder composition layer being smaller than the amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

Moreover, yet another manufacturing method according to this embodiment is:

a method for manufacturing a layered body, the method including a step of sintering a calcined body at 1200°C or above and 1600°C or below,

the calcined body having a surface zirconia composition layer containing zirconia that contains at least one stabilizing element and also has a necking structure and a composition-gradient zirconia composition layer composed of two or more unit zirconia composition layers,

the unit zirconia composition layers each containing at least one coloring element and zirconia that contains at least one stabilizing element and also has a necking structure,

the composition-gradient zirconia composition layer being constructed as a result of the unit zirconia composition layers being stacked in such a manner that the amount of the stabilizing element in the zirconia containing at least one stabilizing element contained in the composition-gradient zirconia composition layer remains unchanged or decreases from the surface zirconia composition layer toward the surface of the calcined body opposite the surface zirconia composition layer, and

the amount of the stabilizing element in the zirconia containing at least one stabilizing element contained in the surface zirconia composition layer being smaller than the amount of the stabilizing element in the zirconia containing at least one stabilizing element contained in a first composition-gradient zirconia composition layer, which is one of the unit zirconia composition layers constituting the composition-gradient zirconia composition layer and is adjacent to the surface zirconia composition layer.

A manufacturing method in the configuration in which the layered body according to this embodiment is a calcined body is:

a method for manufacturing a layered body, the method comprising a step of calcining a green body at 800°C or above and below 1200°C to turn the green body into a calcined body,

the green body having a surface powder composition layer containing zirconia containing at least one stabilizing element and a composition-gradient powder composition layer composed of two or more unit powder composition layers,

the unit powder composition layers each containing at least one coloring element and zirconia containing at least one stabilizing element,

the composition-gradient powder composition layer being constructed as a result of the unit powder composition layers being stacked in such a manner that the amount of the stabilizing element in the zirconia containing at least one stabilizing element contained in the composition-gradient powder composition layer remains unchanged or decreases from the surface powder composition layer toward the surface powder composition of the green body opposite the surface powder composition layer, and

the amount of the stabilizing element in the zirconia containing at least one stabilizing element contained in the surface powder composition layer being smaller than the amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

(Green Body)

For the green body used for the manufacturing methods according to this embodiment, major differences from the sintered body and calcined body described above will be described in the following.

The green body used for the manufacturing methods according to this embodiment is:

a green body having a surface powder composition layer containing zirconia containing at least one stabilizing element and a composition-gradient powder composition layer composed of two or more unit powder composition layers,

the unit powder composition layers each containing at least one coloring element and zirconia containing at least one stabilizing element,

the composition-gradient powder composition layer being constructed as a result of the unit powder composition layers being stacked in such a manner that the amount of the stabilizing element in the zirconia containing at least one stabilizing element contained in the composition-gradient powder composition layer remains unchanged or decreases from the surface powder composition layer toward the surface powder composition of the green body opposite the surface powder composition layer, and

the amount of the stabilizing element in the zirconia containing at least one stabilizing element contained in the surface powder composition layer being smaller than the amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

The green body is a composition having a multilayer structure, or is a so-called layered body, and is a layered body made of powder compositions. The green body can be used as a precursor to a calcined body or sintered body.

The green body has powder composition layers made of powder compositions containing zirconia containing at least one stabilizing element (Hereinafter also referred to as "powder layers.") instead of having zirconia layers, and has a structure equivalent to the layered body structure described with the sintered body. The green body, therefore, can also be considered a layered body including three or more layers containing a powder of zirconia containing at least one stabilizing element. In the green body, the stabilizing element may be in a state in which it is dissolved in the zirconia or may be in a state of an oxide or at least one precursor to it. An example for the precursor is one or more selected from the group of a sulfide, a chloride, a nitrate, a sulfate, an hydroxide and an oxyhydroxide or even one or more selected from the group of a chloride, a hydroxide and an oxyhydroxide. It should be noted that the surface powder composition layer may contain at least one coloring element. When the surface powder composition layer contains at least one coloring element, it is preferred that the coloring element include at least an oxide.

For the green body, it is preferred that the warpage be 1.0 mm or less, more preferably 0.3 mm or less, even more preferably 0.2 mm or less, still more preferably 0.1 mm or less, still more preferably 0.05 mm or less. Although it is preferred that the green body have no warpage (the warpage be 0 mm), the green body may have an extent of warpage that cannot be measured with gauges (the warpage may be 0 mm or more). The green body can have a warpage of, for example, greater than 0 mm or even 0.01 mm or more. The warpage is preferably 0.06 mm or less, 0.05 mm or less or even below the measurement limit (less than 0.03 mm).

For the green body, it is preferred that the amount of deformation be 1.0 or less, more preferably 0.5 or less, even more preferably 0.2 or less, still more preferably 0.15 or less. The amount of deformation can be, for example, 0 or greater, 0.01 or greater or even 0.05 or greater.

The zirconia contained in the powder layers is preferably zirconia resulting from the heat treatment of zirconia sol, more preferably zirconia resulting from the heat treatment of zirconia sol obtained via the hydrolysis of a zirconium compound, even more preferably zirconia resulting from the heat treatment of zirconia sol produced through the hydrolysis of zirconium oxychloride.

The zirconia contained in the powder layers is preferably zirconia powder. For the zirconia powder, it is preferred that the average particle size be 0.3 um or more and 0.7 um or less, preferably 0.4 um or more and 0.5 um or less.

For the zirconia powder, it would be beneficial that the BET specific surface area be 7.5 $m^2$/g or more and 15 $m^2$/g or less. When the BET specific surface area falls below this range, the sintering rate is too slow, and when the BET specific surface area exceeds this range, by contrast, the sintering rate is too fast. In either case, densification is impeded in sintering under atmospheric pressure, particularly in sintering in the air. Since densification tends to be promoted even when sintering at a high temperature increase rate is applied, the lower limit to the BET specific surface area is preferably 8 $m^2$/g or more, 9 $m^2$/g or more or 9.5 $m^2$/g or more, and the upper limit to the BET specific surface area is preferably 15 $m^2$/g or less, 13 $m^2$/g or less or 11 $m^2$/g or less. These upper and lower limits may be in any combination. Consequently, the BET specific surface area of the zirconia powder is preferably, for example, 8 $m^2$/g or more and 15 $m^2$/g or less, 9 $m^2$/g or more and 13 $m^2$/g or less or 9.5 $m^2$/g or more and 11 $m^2$/g or less.

In this embodiment, the BET specific surface area is the BET specific surface area as measured as per JIS R 1626 and can be measured by the BET five-point method using a carrier gas method in which nitrogen is used as the adsorbate gas. An example of specific conditions for the measurement of the BET specific surface area is the following conditions.

[Conditions for the Measurement of the BET Specific Surface Area]

Adsorbate medium: $N_2$
Adsorption temperature: -196°C
Pretreatment conditions: Degassing treatment for 1 hour or longer at 250°C in an air atmosphere

The BET specific surface area can be measured using a common instrument (e.g., TriStar II 3020, manufactured by Shimadzu Corporation).

The coloring element contained in the powder layers is preferably in a state in which it is mixed with a zirconia powder, a state in which it is dissolved in the zirconia or both. It may be, furthermore, in a state in which coloringelement and zirconia powders are mixed together.

The green body may contain at least one binder. When containing a binder, the green body has improved shape retainability. The binder contained in the green body can be known one(s) used in the shaping of ceramics, preferably is at least one organic binder. The organic binder is one or more types selected from the group of polyvinyl alcohol, polyvinyl butyrate, waxes and acrylic resins, preferably one or more types of polyvinyl alcohol and acrylic resins, more preferably at least one acrylic resin. In this embodiment, acrylic resins are polymers containing at least one of an acrylate or methacrylate. Specific examples of acrylic resins include one or more types selected from the group of polyacrylic acid, polymethacrylic acid, acrylic acid copolymers and methacrylic acid copolymers and derivative(s) of them.

The amounts of the stabilizing element and the coloring element in the green body and each powder layer can be freely chosen; however, they can be any percentages similar to those in the sintered body according to this embodiment described above.

When each powder layer contains a binder, it is more preferred that the lower limit, as an amount of the binder in each powder layer, be 1.5% by mass or more, even more preferably 2.0% by mass or more, still more preferably 2.5% by mass or more, and it is preferred that the upper limit be 8.0% by mass or less, more preferably 6.0% by mass or less, even more preferably 5.5% by mass or less, for the reduction of defects during shaping. These upper and lower limits may be in any combination. Consequently, the amount of the binder in each powder layer is preferably, for example, 1.5% by mass or more, more preferably 1.5% by mass or more and 8.0% by mass or less, even more preferably 2.0% by mass or more and 6.0% by mass or less, still more preferably 2.5% by mass or more and 5.5% by mass or less.

The amount of the binder is the percentage by mass of the binder in relation to the mass of the powder composition in the powder layer excluding the binder ({binder/(powder composition - binder)} $\times$ 100), and an example is to determine the total mass of the constituents of the powder composition other than the binder (e.g., the stabilizing element converted into an oxide, zirconia and alumina) and then the percentage by mass of the binder of interest in relation to it before producing the powder composition and produce the powder composition thereafter.

In order for the warpage of the green body to be reduced, the amount of the binder in each powder layer is preferably adjusted according to the amount of the stabilizing element in the adjacent powder layer(s).

From the viewpoint of ease of handling, the powder compositions contained in the powder layers are preferably powders in a state in which zirconia powder, the coloring element and, if a binder is contained, the binder have been granulated (Hereinafter also referred to as "granulated powders."), more preferably granulated powders that have been granulated into granule form through spray drying or a similar process (Hereinafter also referred to as "powder granules.").

The particle sizes of the granulated powders can be freely chosen, but the lower limit can be, for example, 1 um or more, preferably 5 um or more, as an average aggregate size (Hereinafter also referred to as "average granule size."), and the upper limit can be, for example, 150 um or less, preferably 100 um or less, more preferably 50 um or less, even more preferably 30 um or less. These upper and lower limits may be in any combination. Consequently, the particle sizes of the granulated powders can be, for example, 1 um or more and 150 um or less, preferably 1 um or more and 100 um or less, more preferably 5 um or more and 50 um or less, even more preferably 5 um or more and 30 um or less as average aggregate sizes. An example of another embodiment is 20 um or more and 50 um or less.

In this embodiment, the average aggregate size is a size corresponding to a cumulative percentage of 50% in the measurement of particle size distribution by volume. The particle size distribution represents values that can be measured using a common instrument (e.g., MT3100II, manufactured by MicrotracBEL Corporation), representing volume sizes of the particles approximated as spheres.

As a pretreatment, the granulated powders can be sieved using a sieve with an opening of 125 um prior to the measurement.

The green body preferably includes at least a powder layer containing zirconia in which the tetragonal or cubic phase is its primary phase.

For the green body, the density can be, for example, 2.4 g/cm$^3$ or more, preferably 3.1 g/cm$^3$ or more, and can be 3.7 g/cm$^3$ or less, preferably 3.5 g/cm$^3$ or less. Densities within these ranges correspond to 40% to 60% for relative densities.

The density of the green body can be determined from the mass, which can be determined by mass measurement, and the volume, which can be determined by dimensional measurement.

The color of the zirconia layers included in the green body may be different from that of a sintered body obtained by sintering the green body, or there may be no changes in color.

The green body and each powder layer are nontransparent and have a total transmittance of 0%. When measurement errors are considered, however, the total transmittance can be, for example, 0% or more and 0.2% or less.

The green body can have any level of strength such that breakage and chipping when the green body is used during calcination or sintering will not occur.

The green body can be obtained by stacking layers of powder compositions and shaping them. Each powder composition can be obtained by mixing zirconia powder and, if a binder is contained, the binder in any desired proportions by a known method. The shaping process is preferably compression molding. For example, the powder composition having a chemical makeup corresponding to the back layer is introduced into a shaping mold and shaped into the back

EP 4 480 936 A1

layer. Then the powder composition having a chemical makeup corresponding to the chemical makeup of the layer adjacent to the back layer is introduced onto the back layer. When a green body having a structure in which three or more powder layers are stacked together is to be formed, similar operations can be repeated to stack required layers of powder compositions. After the powder composition having a chemical makeup corresponding to the chemical makeup of the surface layer is introduced, uniaxial compression molding is performed at any pressure to give a preliminary green body. By subjecting it to cold isostatic pressing (Hereinafter also referred to as "CIP.") treatment, the green body is obtained. During the stacking process, there is no need to apply vibrations that would form mixed layers between the layers, such as vibrations produced using vibratory equipment. The uniaxial compression molding, furthermore, is preferably performed after the introduction of the powder composition having a chemical makeup corresponding to the surface layer; it is preferred not to perform compression before introducing the powder composition having a chemical makeup corresponding to the surface layer.

The shaping pressure in the uniaxial compression molding is preferably 15 MPa or more, more preferably 18 MPa or more, and preferably is 200 MPa or less, more preferably 100 MPa or less. In uniaxial compression molding, the warpage of the green body tends to be reduced with increasing shaping pressure. As for the pressure in the CIP treatment, an example is a shaping pressure of 98 MPa or more and 392 MPa or less.

Treating the green body at a temperature below its sintering temperature turns the green body into a calcined body. The calcination method and calcination conditions can be known methods.

The temperature at which the green body is held during the calcination process (Hereinafter also referred to as "the calcination temperature.") can be, for example, 800°C or above and below 1200°C, preferably 900°C or above and 1150°C or below, more preferably 950°C or above and 1100°C or below.

The duration for which the green body is held at the calcination temperature (Hereinafter also referred to as "the duration of calcination.") is, for example, preferably 0.5 hours or more and 5 hours or less, more preferably 0.5 hours or more and 3 hours or less.

It should be noted that the temperature elevation may be performed with the elevated temperature, the rate of temperature increase during the elevation and the duration for which the green body is held at the elevated temperature divided into multiple stages.

For example, the green body may be calcined under conditions like 15°C/hour from room temperature to 300°C, holding at 300°C for 5 hours, 15°C/hour from 300°C to 700°C, holding at 700°C for 1 hour, 50°C/hour from 700°C to 1000°C and holding at 1000°C for 2 hours.

The atmosphere(s) during the calcination step (Hereinafter also referred to as "the calcination atmosphere(s).") is preferably at least one atmosphere other than a reducing atmosphere, more preferably at least one of an oxygen atmosphere or air atmosphere, even more preferably an air atmosphere.

In the manufacturing methods according to this embodiment, one of a green body or calcined body (Hereinafter, these are also collectively referred to as "the green body, etc.") is processed at 1200°C or above and 1600°C or below. Through this, the green body, etc., turn into sintered bodies. Before the sintering process, the green body, etc., may be worked into any shape.

The sintering method(s) and sintering conditions can be known methods. An example for the sintering method(s) is at least one type selected from the group of sintering under atmospheric pressure, HIP treatment, SPS and vacuum sintering. The sintering method(s) is preferably sintering under atmospheric pressure, more preferably sintering under atmospheric pressure in an air atmosphere, because these are used versatilely as industrial sintering methods. The sintering method(s) is preferably sintering under atmospheric pressure alone, and it is more preferred not to perform sintering under applied pressure after the sintering under atmospheric pressure. This allows the sintered body to be obtained as a sintered body sintered under atmospheric pressure. In this embodiment, sintering under atmospheric pressure is a method in which during sintering, the article that is to be sintered is sintered by simply heating it, without applying external force.

For the temperature at which the green body, etc., are held during the sintering process (Hereinafter also referred to as "the sintering temperature."), the lower limit is 1200°C or above, preferably 1300°C or above, more preferably 1400°C or above, even more preferably 1430°C or above, still more preferably 1480°C or above, and the upper limit is 1650°C or below, preferably 1580°C or below, more preferably 1560°C or below, even more preferably 1560°C or below, still more preferably 1560°C or below. These upper and lower limits may be in any combination. Consequently, the sintering temperature is, for example, 1200°C or above and 1650°C or below, preferably 1300°C or above and 1580°C or below, more preferably 1400°C or above and 1560°C or below, even more preferably 1430°C or above and 1560°C or below, still more preferably 1480°C or above and 1560°C or below. In another embodiment, the sintering temperature is 1450°C or above and 1650°C or below, preferably 1500°C or above and 1650°C or below, more preferably 1550°C or above and 1650°C or below.

As for the rate of temperature increase to the sintering temperature, the lower limit is 50°C/hour or more for example, preferably 100°C/hour or more, more preferably 150°C/hour or more, and the upper limit is 800°C/hour or less for example, preferably 700°C/hour or less. These upper and lower limits may be in any combination. Consequently, the rate of temperature increase to the sintering temperature is, for example, 50°C/hour or more and 800°C/hour or less, preferably 100°C/hour or more and 800°C/hour or less, more preferably 150°C/hour or more and 800°C/hour or less, even more preferably 150°C/hour or more and 700°C/hour or less.

The duration for which the green body, etc., are held at the sintering temperature (Hereinafter also referred to as "the duration of sintering.") varies depending on the sintering temperature. Preferably, however, the lower limit is 1 hour or more, and the upper limit is 5 hours or less, more preferably 3 hours or less, even more preferably 2 hours or less. These upper and lower limits may be in any combination. Consequently, the duration of sintering is, for example, preferably 1 hour or more and 5 hours or less, more preferably 1 hour or more and 3 hours or less, even more preferably 1 hour or more and 2 hours or less, although it varies depending on the sintering temperature.

For example, the green body, etc., may be sintered under conditions like 100°C/hour from room temperature to 1500°C and holding at 1500°C for 2 hours.

The atmosphere(s) for sintering (Hereinafter also referred to as "the sintering atmosphere(s).") is preferably at least one atmosphere other than a reducing atmosphere, more preferably at least one of an oxygen atmosphere or air atmosphere, even more preferably an air atmosphere. An example of an air atmosphere is being composed primarily of nitrogen and oxygen and having an oxygen concentration of approximately 18% to 23% by volume.

An example of preferred sintering conditions in the sintering step is sintering under atmospheric pressure in an air atmosphere.

[EXAMPLES]

The layered body according to the present disclosure will now be described using examples. The present disclosure, however, is not limited to these examples.

(BET Specific Surface Area)

The BET specific surface area was measured by the BET five-point method as per JIS R 1626 using an automatic specific surface area automatic analyzer (analyzer name, TriStar II 3020; manufactured by Shimadzu Corporation). The measurement conditions are as follows.

Adsorbate medium: $N_2$
Adsorption temperature: -196°C
Pretreatment conditions: Degassing treatment for 1 hour or longer at 250°C in an air atmosphere

(Average Granule Size)

The average granule size was measured by particle size distribution measurement by laser diffraction and scattering using a Microtrac particle size analyzer (analyzer name, MT3100II; manufactured by MicrotracBEL Corporation). The measurement conditions are as follows.

Light source: Semiconductor laser (wavelength: 780 nm)
Voltage: 3 mW
Refractive index of zirconia: 2.17
Calculation mode: MT3000

As a pretreatment, a granulated powder was sieved using a sieve with an opening of 125 um prior to the measurement. The granulated powder that passed through the sieve was used as the sample for measurement.

(Warpage and the Amount of Deformation)

With a layered body in a disk shape (a green body, calcined body or sintered body) as the sample for measurement, the amount of deformation was determined from equation (3) below.

$$\text{The amount of deformation} = (\text{warpage: mm})/(\text{dimension: mm}) \times 100 \quad \dots \quad (3)$$

How to determine the amount of deformation will be described in further detail.
The conditions for the preparation of the layered body are as follows.

[Layered body]

Mold diameter: Φ110 mm
Powder mass: 470 g (total mass of the powders used in the layered body)
Shaping pressure: Uniaxial compression molding, 49 MPa + CIP treatment, 196 MPa
Shaping steps: Introduce the raw material powder that will form the first layer into the mold → Level the powder → Introduce the raw material powder that will form the second layer into the mold → Level the powder → (Repeat) → Uniaxial compression molding → CIP treatment

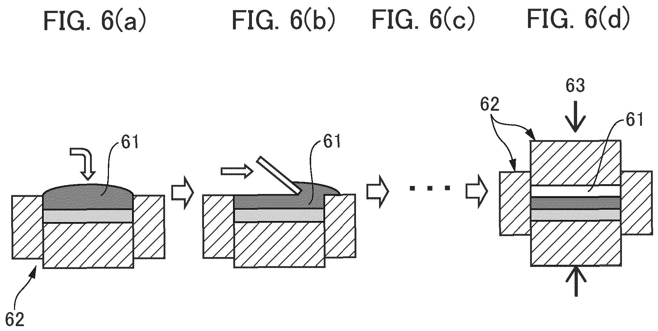

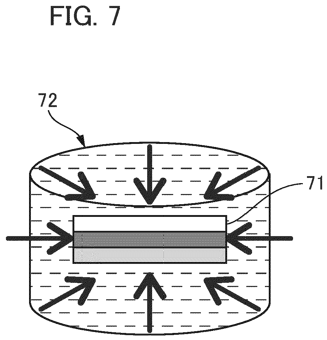

A diagram schematically representing the shaping step of uniaxial compression molding, during the preparation of a layered body described above, is presented in Fig. 6, and a diagram schematically representing the shaping step of CIP treatment is presented in Fig. 7.

In Fig. 6, it is illustrated how powder compositions are compressed by (a) introducing a powder composition (61) into a mold (62) → (b) leveling the powder composition → (c) (forming the second layer in the same manner and repeating the operations) and (d) uniaxial compression molding (63).

In Fig. 7, it is illustrated how the uniaxially compressed powder (layered body) (71) is placed in a high-pressure vessel, the inside of the high-pressure vessel is filled with a solvent (72), and the powder is isotropically compressed.

Through the CIP treatment, a green body for the measurement of warpage and the amount of deformation was obtained.

This green body was subjected to calcination under the conditions below, through which a calcined body for the measurement of warpage and the amount of deformation was obtained.

The above green body was subjected to calcination and sintering under the conditions below, through which a sintered body for the measurement of warpage and the amount of deformation was obtained.

[Calcination and Sintering Conditions]

Calcination: 15°C/hour from room temperature to 300°C, holding at 300°C for 5 hours, 15°C/hour from 300°C to 700°C, holding at 700°C for 1 hour, 50°C/hour from 700°C to 1000°C and holding at 1000°C for 2 hours, followed by cooling inside the furnace.

Sintering: 100°C/hour from room temperature to 1500°C and holding at 1500°C for 2 hours, followed by cooling inside the furnace.

For the specific types of layered bodies, i.e., the green body, calcined body and sintered body, the measurement of warpage was performed by the measurement method illustrated in Fig. 3. The sample for measurement was positioned in such a manner that the outwardly curved portion of the sample for measurement was in contact with a horizontal plate. By inserting thickness gauges conforming to JIS B 7524: 2008 (product name, 75A19; manufactured by K.K. Nagai Gauge Seisakusho) into the gap created between the horizontal plate and the bottom surface, the warpage was measured. The measurement was performed by inserting one or more gauges positioned parallel to the top of the horizontal plate into the gap created between the horizontal plate and the bottom surface of the sample for measurement and measuring the gauge thickness that was the maximum thickness of gauges that were successfully inserted into the gap, and this gauge thickness was reported as the warpage. It should be noted that the warpage was measured sequentially in 0.01 mm steps starting from a gauge thickness of 0.03 mm by using a single gauge or a combination of gauges.

As for the dimension of the sample for measurement, the diameter at the upper end and the diameter at the lower end were measured at four points each using a caliper, and the average of the diameters at the upper and lower ends was determined.

Synthesis Example 1 (synthesis of zirconia powder)

(Zirconia Powder A1)

An aqueous solution of zirconium oxychloride was subjected to a hydrolysis reaction to give hydrated zirconia sol. After yttrium chloride and erbium oxide were added to this hydrated zirconia sol to achieve an yttrium concentration of 5.09 mol% on an $Y_2O_3$ basis and an erbium concentration of 0.07 mol% on an $Er_2O_3$ basis, the resulting mixture was dried at 180°C. After the dried zirconia sol was fired at 1175°C for 2 hours, the product was waterwashed with purified water and dried at 110°C in an air atmosphere, $\alpha$-alumina, iron oxide as an oxide coloring element and purified water were mixed into the product to give a slurry, and this slurry was processed in a ball mill for 22 hours to give a slurry containing 0.05% by mass of alumina and 0.06% by mass, on an iron oxide basis, of iron as an oxide coloring element with the remainder being zirconia containing 5.09 mol% yttrium and 0.07 mol% erbium. For the resulting slurry, an acrylic acid binder was added to and mixed with the slurry in such a manner that the percentage by mass of the binder to the mass of powder in the slurry would be 3% by mass. By spray-drying this slurry at 180°C in an air atmosphere, zirconia powder A1 was obtained. The zirconia powder obtained had a BET specific surface area of 10.1 $m^2$/g and an average granule diameter of 45 um. The composition of zirconia powder A1 is presented in Table 1 below.

Zirconia powder A1 was subjected to uniaxial compression molding at a pressure of 49 MPa and CIP treatment at a pressure of 196 MPa to give a powder layer of zirconia powder A1.

By calcining and sintering the resulting powder layer under the following conditions, a sintered body layer was obtained.

[Calcination and Sintering Conditions]

Calcination: 15°C/hour from room temperature to 300°C, holding at 300°C for 5 hours, 15°C/hour from 300°C to 700°C, holding at 700°C for 1 hour, 50°C/hour from 700°C to 1000°C and holding at 1000°C for 2 hours, followed by cooling inside the furnace.

Sintering: 100°C/hour from room temperature to 1500°C and holding at 1500°C for 2 hours, followed by cooling inside the furnace.

For the sintered body layer of zirconia powder A1, the three-point flexural strength (MPa), total transmittance (%) and color (L*, a* and b*) were measured by the methods described below.

(Three-Point Flexural Strength)

The measurement of the three-point flexural strength was measured by a method according to JIS R 1601, illustrated in Fig. 4, as mentioned above.

(Total Transmittance)

The total transmittance of a sample was measured by a method based on the method of JIS K 7361. The total transmittance was measured by illuminating a sample for measurement with standard illuminant D65 and detecting the beam of light that passed through the sample for measurement with an integrating sphere. In the measurement, a common haze meter (instrument name, NDH4000 haze meter; manufactured by NIPPON DENSHOKU) was used.

A sample in a disk shape was used as the sample for measurement. Prior to the measurement, a certain point of the sintered body was cut out horizontally, and both sides of this sample were mirror-polished, achieving a sample thickness of 1.0 ± 0.1 mm and a surface roughness (Ra) of 0.02 um or less.

(Color L*, a*, b*, ∆L* and ∆C*)

The color of the sintered body was measured using a spectrocolorimeter equipped with illuminating and receiving optical systems conforming to geometric conditions c in JIS Z 8722 (instrument name, CM-700d; manufactured by Konica Minolta, Inc.). The measurement conditions are as follows.

Light source: D65 light source
Viewing angle: 10°
Measurement method: SCI
Background: Black board

After a certain point of the sintered body was cut out in the horizontal direction as the sample for measurement, both sides of this sample were mirror-polished, achieving a diameter of 20 mm × a thickness of 1.0 ± 0.1 mm and a surface roughness (Ra) of 0.02 um or less. The sample for measurement was placed on a black board, and, with both polished surfaces as the surfaces for evaluation, the color (L*, a* and b*) of each surface was measured (black background measurement). The absolute difference in measured L* between the surfaces was determined as ∆L*, and the absolute difference in C*, determined from a* and b*, was determined as ∆C*. As the effective area for color evaluation, a diameter of 10 mm was employed.

The results of the evaluation of characteristics for the sintered body made with zirconia powder A1 are presented in Tables 1 and 2.

(Zirconia Powder A2)

Zirconia powder A2, having the composition in Table 1, was prepared by the same method as zirconia powder A1, except that yttrium chloride and erbium oxide were added to achieve an yttrium concentration of 5.14 mol% on an $Y_2O_3$ basis and an erbium concentration of 0.03 mol% on an $Er_2O_3$ basis and that iron oxide and cobalt oxide were used as oxide coloring elements with their amounts changed as specified in Table 1.

By the same method as with zirconia powder A1, a sintered body layer made with zirconia powder A2 was prepared, and the characteristics of this sintered body layer were evaluated. The evaluation results are presented in Tables 1 and 2.

(Zirconia Powder A3)

Zirconia powder A3, having the composition in Table 1, was prepared by the same method as zirconia powder A1, except that no alumina was used.

By the same method as with zirconia powder A1, a sintered body layer made with zirconia powder A3 was prepared, and the characteristics of this sintered body layer were evaluated. The evaluation results are presented in Tables 1 and 2.

(Zirconia Powders B1 to B3 and C1 to C10)

Zirconia powders B1 to B3 and C1 to C10, having the compositions in Table 1, were prepared by the same method as zirconia powder A1, except that compared to zirconia powder A1, the types and amounts of stabilizing elements and the types and amounts of coloring elements were changed as specified in Table 1.

For zirconia powders B1 to B3 and C1 to C10, too, sintered body layers made with these powders were prepared, and the characteristics of these sintered body layers were evaluated. The evaluation results are presented in Tables 1 and 2.

[Table 1]

| Powder number | Powder characteristics | | | | | | | | | | Characteristics of the green body layer | Characteristics of the sintered body layer | | | | |
|---|---|---|---|---|---|---|---|---|---|---|---|---|---|---|---|---|
| | Stabilizing elements (mol%) | | | $Al_2O_3$ (wt%) | Coloring elements (wt%) | | | | BET specific surface area (m²/g) | Average granule size (µm) | Density (g/cm³) | Density (g/cm³) | Color | | | |
| | $Y_2O_3$ | $Er_2O_3$ | Y+Er | | $Er_2O_3$ | $Fe_2O_3$ | $Co_3O_4$ | $TiO_2$ | | | | | L* | a* | b* | C* |
| A1 | 5.09 | 0.07 | 5.16 | 0.05 | 0.20 | 0.06 | - | - | 10.1 | 45 | 3.31 | 6.06 | 74.6 | -2.4 | 7.9 | 8.3 |
| A2 | 5.14 | 0.03 | 5.18 | 0.05 | 0.10 | 0.14 | 0.01 | - | 10.2 | 44 | 3.32 | 6.05 | 68.6 | -1.1 | 12.1 | 12.2 |
| A3 | 5.09 | 0.07 | 5.16 | 0.00 | 0.20 | 0.06 | - | - | 10.5 | 44 | 3.31 | 6.06 | 73.8 | -2.5 | 8.1 | 8.5 |
| B1 | 5.38 | 0.07 | 5.45 | 0.05 | 0.20 | 0.06 | - | - | 10.1 | 44 | 3.31 | 6.05 | 74.6 | -2.6 | 7.9 | 8.3 |
| B2 | 5.26 | 0.14 | 5.40 | 0.05 | 0.40 | 0.09 | - | - | 10.1 | 44 | 3.31 | 6.06 | 73.1 | -1.6 | 11.3 | 11.4 |
| B3 | 5.44 | 0.03 | 5.48 | 0.05 | 0.10 | 0.14 | 0.01 | - | 10.1 | 44 | 3.32 | 6.05 | 68.7 | -1.2 | 12.1 | 12.1 |
| C1 | 3.93 | 0.06 | 3.99 | 0.05 | 0.17 | 0.05 | - | - | 10.1 | 44 | 3.31 | 6.08 | 76.8 | -1.9 | 10.0 | 10.2 |
| C2 | 3.85 | 0.12 | 3.97 | 0.05 | 0.34 | 0.08 | - | - | 10.1 | 44 | 3.31 | 6.08 | 74.9 | -0.5 | 13.7 | 13.7 |
| C3 | 3.80 | 0.16 | 3.96 | 0.05 | 0.46 | 0.11 | - | - | 10.1 | 44 | 3.32 | 6.08 | 71.7 | 1.9 | 17.9 | 18.0 |
| C4 | 3.89 | 0.03 | 3.93 | 0.05 | 0.10 | 0.05 | - | - | 10.1 | 44 | 3.31 | 6.08 | 74.9 | -1.1 | 6.8 | 6.8 |
| C5 | 3.89 | 0.00 | 3.89 | 0.05 | 0.00 | 0.09 | - | - | 10.1 | 44 | 3.32 | 6.07 | 71.6 | -0.7 | 12.5 | 12.5 |
| C6 | 3.80 | 0.03 | 3.84 | 0.05 | 0.10 | 0.13 | 0.01 | - | 10.1 | 44 | 3.32 | 6.08 | 69.1 | 1.3 | 14.3 | 14.4 |
| C7 | 3.89 | 0.07 | 3.96 | 0.05 | 0.20 | 0.05 | - | 0.23 | 10.2 | 44 | 3.30 | 6.08 | 77.6 | -1.8 | 10.3 | 10.5 |
| C8 | 3.82 | 0.15 | 3.97 | 0.05 | 0.45 | 0.08 | - | 0.34 | 10.4 | 44 | 3.30 | 6.08 | 75.7 | -0.4 | 13.8 | 13.8 |
| C9 | 3.89 | 0.07 | 3.96 | 0.05 | 0.20 | 0.05 | - | - | 10.3 | 44 | 3.30 | 6.08 | 76.4 | -1.6 | 10.1 | 10.2 |
| C10 | 3.82 | 0.15 | 3.97 | 0.05 | 0.45 | 0.07 | - | - | 10.4 | 44 | 3.30 | 6.08 | 75.4 | -0.2 | 13.4 | 13.4 |

EXAMPLE 1

(Green Body)

After 141.0 g of zirconia powder C6 was introduced into a mold having an inner diameter of 110 mm, the mold was subjected to tapping to make a fourth powder layer (corresponding to a second composition-gradient powder composition layer). 98.7 g of zirconia powder C2 was introduced onto the fourth powder layer, and the mold was subjected to tapping to make a third powder layer (corresponding to a third composition-gradient powder composition layer). 89.3 g of zirconia powder B1 was introduced onto the third powder layer, and the mold was subjected to tapping to make a second powder layer (corresponding to the first composition-gradient composition layer). After 141.0 g of zirconia powder A1 was introduced onto the second powder layer and the mold was subjected to tapping to make a first powder layer (corresponding to the surface powder composition layer), uniaxial compression molding was performed at a pressure of 98 MPa. Thereafter, the workpiece was subjected to CIP treatment at a pressure of 196 MPa to give a layered body consisting of four layers, and this layered body was designated as the green body in this example.

(Calcined Body)

The green body was calcined to give a layered body by subjecting it to 15°C/hour from room temperature to 300°C, holding at 300°C for 5 hours, 15°C/hour from 300°C to 700°C, holding at 700°C for 1 hour, 50°C/hour from 700°C to 1000°C and holding at 1000°C for 2 hours and subsequent cooling inside the furnace, and the layered body was designated as the calcined body in this example. The relative thickness in the calcined body for each layer is presented in Table 2.

(Sintered Body)

The calcined body was fired with a rate of temperature increase of 100°C/hour, a sintering temperature of 1500°C and a duration of sintering of 2 hours to give a layered body, and this layered body was designated as the sintered body in this example.

The results of the measurement of warpage and the amount of deformation for the green body, calcined body and sintered body are presented in Table 2 below. The result of the measurement of the warpage of the sintered body in Example 1 was below the measurement limit (0.03 mm).

(EXAMPLES 2 to 15 and COMPARATIVE EXAMPLE 1)

The layered bodies specified in Table 2 were produced by the same method as in Example 1, except that compared to Example 1, the types of powders used, the powder masses and the order of stacking of the powders used were changed as specified in Table 2. For the green body, calcined body and sintered body in each example, warpage and the amount of deformation were measured. The measurement results are presented in Table 2. It should be noted that in Table 2, "N.D." indicated as the result of the measurement of warpage means that the result was below the measurement limit, "less than 0.03 mm." In Table 2, furthermore, $\Delta L^*$ and $\Delta C^*$ are differences in lightness and chroma, respectively, between unit zirconia layers constituting the composition-gradient zirconia layer. For instance, in Example 1, these values indicate that $\Delta L^*$ and $\Delta C^*$ between the first composition-gradient zirconia layer, obtained from powder B1, and the third composition-gradient zirconia layer, obtained from powder C2, are 5.8 and 5.4, respectively, and that $\Delta L^*$ and $\Delta C^*$ between the third composition-gradient zirconia layer, obtained from powder C2, and the second composition-gradient zirconia layer, obtained from powder C6, are 0.7 and 0.7, respectively.

[Table 2]

| | Powder number | Powder characteristics | | | | Conditions | | Characteristics of the sintered body layer | | | | | | Characteristics of the layered bodies | | | | | |
|---|---|---|---|---|---|---|---|---|---|---|---|---|---|---|---|---|---|---|---|
| | | Stabilizing elements (mol%) | | | | Relative thickness in the calcined body | Powder mass | Three-point flexural strength | Total transmittance | ΔL* | ΔC* | Warpage (mm) | | | Amount of deformation | | | | |
| | | Y₂O₃ | Er₂O₃ | Y+Er | Second - first powder layer | | (g) | (MPa) | (%) | | | Green | Calcined | Sintered | Green | Calcined | Sintered | | |
| Example 1 | A1 | 5.09 | 0.07 | 5.16 | 0.29 | 30 | 141.0 | 807 | 44.2 | 5.8 0.7 | 5.4 0.7 | 0.04 | 0.22 | 0.08 | 0.04 | 0.21 | 0.09 | | |
| | B1 | 5.38 | 0.07 | 5.45 | | 19 | 89.3 | 611 | 44.2 | | | | | | | | | | |
| | C2 | 3.85 | 0.12 | 3.97 | | 21 | 98.7 | 1095 | 37.7 | | | | | | | | | | |
| | C6 | 3.80 | 0.03 | 3.84 | | 30 | 141.0 | 1101 | 26.0 | | | | | | | | | | |
| Example 2 | A1 | 5.09 | 0.07 | 5.16 | 0.29 | 30 | 141.0 | 807 | 44.2 | 5.8 0.7 | 5.4 0.7 | 0.04 | 0.22 | 0.08 | 0.04 | 0.21 | 0.09 | | |
| | B1 | 5.38 | 0.07 | 5.45 | | 21 | 98.7 | 611 | 44.2 | | | | | | | | | | |
| | C2 | 3.85 | 0.12 | 3.97 | | 19 | 89.3 | 1095 | 37.7 | | | | | | | | | | |
| | C6 | 3.80 | 0.03 | 3.84 | | 30 | 141.0 | 1101 | 26.0 | | | | | | | | | | |
| Example 3 | A1 | 5.09 | 0.07 | 5.16 | 0.29 | 20 | 94.0 | 807 | 44.2 | 2.2 3.7 0.7 | 1.9 3.5 0.7 | N.D. | 0.17 | 0.06 | N.D. | 0.16 | 0.07 | | |
| | B1 | 5.38 | 0.07 | 5.45 | | 20 | 94.0 | 611 | 44.2 | | | | | | | | | | |
| | C1 | 3.93 | 0.06 | 3.99 | | 20 | 94.0 | 1093 | 40.3 | | | | | | | | | | |
| | C2 | 3.85 | 0.12 | 3.97 | | 20 | 94.0 | 1095 | 37.7 | | | | | | | | | | |
| | C6 | 3.80 | 0.03 | 3.84 | | 20 | 94.0 | 1101 | 26.0 | | | | | | | | | | |
| Example 4 | A1 | 5.09 | 0.07 | 5.16 | 0.29 | 30 | 141.0 | 807 | 44.2 | 2.2 3.7 | 1.9 3.5 | N.D. | 0.12 | 0.05 | N.D. | 0.12 | 0.06 | | |
| | B1 | 5.38 | 0.07 | 5.45 | | 19 | 89.3 | 611 | 44.2 | | | | | | | | | | |
| | C1 | 3.93 | 0.06 | 3.99 | | 21 | 98.7 | 1093 | 40.3 | | | | | | | | | | |
| | C2 | 3.85 | 0.12 | 3.97 | | 30 | 141.0 | 1095 | 37.7 | | | | | | | | | | |

| | Powder number | Powder characteristics | | | | Conditions | | Characteristics of the sintered body layer | | ΔL* | ΔC* | Characteristics of the layered bodies | | | | | |
| | | Stabilizing elements (mol%) | | | | Relative thickness in the calcined body | Powder mass | Three-point flexural strength | Total transmittance | | | Warpage (mm) | | | Amount of deformation | | |
| | | $Y_2O_3$ | $Er_2O_3$ | Y+Er | Second - first powder layer | | (g) | (MPa) | (%) | | | Green | Calcined | Sintered | Green | Calcined | Sintered |
| Example 5 | A1 | 5.09 | 0.07 | 5.16 | 0.29 | 30 | 141.0 | 807 | 44.2 | 2.2 / 3.7 | 1.9 / 3.5 | N.D. | 0.12 | 0.05 | N.D. | 0.12 | 0.06 |
| | B1 | 5.38 | 0.07 | 5.45 | | 21 | 98.7 | 611 | 44.2 | | | | | | | | |
| | C1 | 3.93 | 0.06 | 3.99 | | 19 | 89.3 | 1093 | 40.3 | | | | | | | | |
| | C2 | 3.85 | 0.12 | 3.97 | | 30 | 141.0 | 1095 | 37.7 | | | | | | | | |
| Example 6 | A1 | 5.09 | 0.07 | 5.16 | 0.29 | 30 | 141.0 | 807 | 44.2 | 3.4 / 3.1 | 3.1 / 3.0 | 0.03 | 0.18 | 0.04 | 0.03 | 0.17 | 0.05 |
| | B1 | 5.38 | 0.07 | 5.45 | | 21 | 98.7 | 611 | 44.2 | | | | | | | | |
| | B2 | 5.26 | 0.14 | 5.40 | | 19 | 89.3 | 623 | 41.2 | | | | | | | | |
| | C6 | 3.80 | 0.03 | 3.84 | | 30 | 141.0 | 1101 | 26.0 | | | | | | | | |
| Example 7 | A1 | 5.09 | 0.07 | 5.16 | 0.29 | 20 | 94.0 | 807 | 44.2 | 3.4 / 2.4 / 0.7 | 3.1 / 2.3 / 0.7 | N.D. | 0.15 | 0.04 | N.D. | 0.14 | 0.05 |
| | B1 | 5.38 | 0.07 | 5.45 | | 20 | 94.0 | 611 | 44.2 | | | | | | | | |
| | B2 | 5.26 | 0.14 | 5.40 | | 20 | 94.0 | 623 | 41.2 | | | | | | | | |
| | C2 | 3.85 | 0.12 | 3.97 | | 20 | 94.0 | 1095 | 37.7 | | | | | | | | |
| | C6 | 3.80 | 0.03 | 3.84 | | 20 | 94.0 | 1101 | 26.0 | | | | | | | | |
| Example 8 | A1 | 5.09 | 0.07 | 5.16 | 0.24 | 10 | 47.0 | 807 | 44.2 | 4.5 / 7.6 | 4.5 / 7.5 | 0.04 | 0.22 | 0.09 | 0.04 | 0.21 | 0.11 |
| | B2 | 5.26 | 0.14 | 5.40 | | 20 | 94.0 | 623 | 41.2 | | | | | | | | |
| | C4 | 3.89 | 0.03 | 3.93 | | 40 | 188.0 | 1094 | 36.6 | | | | | | | | |
| | C6 | 3.80 | 0.03 | 3.84 | | 30 | 141.0 | 1101 | 26.0 | | | | | | | | |

(continued)

| Example | Powder number | Y₂O₃ (mol%) | Er₂O₃ (mol%) | Y+Er (mol%) | Second-first powder layer (mol%) | Relative thickness in the calcined body | Powder mass (g) | Three-point flexural strength (MPa) | Total transmittance (%) | ΔL* | ΔC* | Warpage Green (mm) | Warpage Calcined (mm) | Warpage Sintered (mm) | Deformation Green | Deformation Calcined | Deformation Sintered |
|---|---|---|---|---|---|---|---|---|---|---|---|---|---|---|---|---|---|
| Example 9 | A2 | 5.14 | 0.03 | 5.18 | | 33 | 155.1 | 804 | 32.0 | | | 0.03 | 0.19 | 0.11 | 0.03 | 0.18 | 0.13 |
| | B3 | 5.44 | 0.03 | 5.48 | 0.30 | 33 | 155.1 | 605 | 32.1 | 2.3 | 2.3 | | | | | | |
| | C6 | 3.80 | 0.03 | 3.84 | | 33 | 155.1 | 1101 | 26.0 | | | | | | | | |
| Example 10 | A1 | 5.09 | 0.07 | 5.16 | | 33 | 155.1 | 807 | 44.2 | | | 0.04 | 0.25 | 0.10 | 0.04 | 0.24 | 0.12 |
| | B2 | 5.26 | 0.14 | 5.40 | 0.24 | 33 | 155.1 | 623 | 41.2 | 1.2 | 1.1 | | | | | | |
| | C5 | 3.89 | 0.00 | 3.89 | | 33 | 155.1 | 1098 | 30.4 | | | | | | | | |
| Example 11 | A1 | 5.09 | 0.07 | 5.16 | | 20 | 94.0 | 807 | 44.2 | 2.2 | 1.9 | 0.04 | 0.11 | 0.10 | 0.04 | 0.10 | 0.12 |
| | B1 | 5.38 | 0.07 | 5.45 | 0.29 | 20 | 94.0 | 611 | 44.2 | | | | | | | | |
| | C1 | 3.93 | 0.06 | 3.99 | | 20 | 94.0 | 1093 | 40.3 | 3.7 | 3.5 | | | | | | |
| | C2 | 3.85 | 0.12 | 3.97 | | 20 | 94.0 | 1095 | 37.7 | 3.7 | | | | | | | |
| | C3 | 3.80 | 0.16 | 3.96 | | 20 | 94.0 | 1005 | 35.6 | 4.2 | 4.3 | | | | | | |
| Example 12 | C1 | 3.93 | 0.06 | 3.99 | | 20 | 94.0 | 1093 | 40.3 | 2.2 | 1.9 | N.D. | 0.06 | 0.05 | N.D. | 0.06 | 0.06 |
| | B1 | 5.38 | 0.07 | 5.45 | 1.46 | 20 | 94.0 | 611 | 44.2 | | | | | | | | |
| | C1 | 3.93 | 0.06 | 3.99 | | 20 | 94.0 | 1093 | 40.3 | 3.7 | 3.5 | | | | | | |
| | C2 | 3.85 | 0.12 | 3.97 | | 20 | 94.0 | 1095 | 37.7 | | | | | | | | |
| Example 13 | A3 | 5.09 | 0.07 | 5.16 | | 30 | 141.0 | 802 | 44.2 | 2.5 | 2.2 | N.D. | N.D. | N.D. | N.D. | N.D. | N.D. |
| | B1 | 5.38 | 0.07 | 5.45 | 0.29 | 19 | 89.3 | 611 | 44.2 | | | | | | | | |
| | C7 | 3.89 | 0.07 | 3.96 | | 21 | 98.7 | 1089 | 40.1 | 3.4 | 3.3 | | | | | | |
| | C8 | 3.82 | 0.15 | 3.97 | | 30 | 141.0 | 1120 | 37.5 | | | | | | | | |

(continued)

| | Powder number | Powder characteristics | | | | Conditions | | Characteristics of the sintered body layer | | | | Characteristics of the layered bodies | | | | | | |
|---|---|---|---|---|---|---|---|---|---|---|---|---|---|---|---|---|---|---|
| | | Stabilizing elements (mol%) | | | | Relative thickness in the calcined body | Powder mass | Three-point flexural strength | Total transmit-tance | $\Delta L^*$ | $\Delta C^*$ | Warpage (mm) | | | Amount of deformation | | | |
| | | $Y_2O_3$ | $Er_2O_3$ | Y+Er | Second - first powder layer | | (g) | (MPa) | (%) | | | Green | Calcined | Sintered | Green | Calcined | Sintered |
| Example 14 | A1 | 5.09 | 0.07 | 5.16 | 0.29 | 30 | 141.0 | 807 | 44.2 | 2.5 3.4 | 2.2 3.3 | N.D. | N.D. | N.D. | N.D. | N.D. | N.D. |
| | B1 | 5.38 | 0.07 | 5.45 | | 19 | 89.3 | 611 | 44.2 | | | | | | | | |
| | C7 | 3.89 | 0.07 | 3.96 | | 21 | 98.7 | 1089 | 40.1 | | | | | | | | |
| | C8 | 3.82 | 0.15 | 3.97 | | 30 | 141.0 | 1120 | 37.5 | | | | | | | | |
| Example 15 | A3 | 5.09 | 0.07 | 5.16 | 0.29 | 30 | 141.0 | 802 | 44.2 | 2.2 3.3 | 2.0 3.1 | N.D. | 0.10 | N.D. | N.D. | 0.10 | N.D. |
| | B1 | 5.38 | 0.07 | 5.45 | | 19 | 89.3 | 611 | 44.2 | | | | | | | | |
| | C9 | 3.89 | 0.07 | 3.96 | | 21 | 98.7 | 1049 | 40.4 | | | | | | | | |
| | C10 | 3.82 | 0.15 | 3.97 | | 30 | 141.0 | 1060 | 37.9 | | | | | | | | |
| Comparative Example 1 | B1 | 5.38 | 0.07 | 5.45 | -1.47 | 30 | 141.0 | 611 | 44.2 | 3.7 4.2 | 3.5 4.3 | 0.05 | 0.24 | 0.13 | 0.05 | 0.23 | 0.15 |
| | C1 | 3.93 | 0.06 | 3.99 | | 19 | 89.3 | 1093 | 40.3 | | | | | | | | |
| | C2 | 3.85 | 0.12 | 3.97 | | 21 | 98.7 | 1095 | 37.7 | | | | | | | | |
| | C3 | 3.80 | 0.16 | 3.96 | | 30 | 141 | 1005 | 35.6 | | | | | | | | |

Examples 1 to 15, in which the stabilizing element content of the surface layer was smaller than the stabilizing element content of the adjacent, first composition-gradient layer, were layered bodies with high mechanical strength; the three-point flexural strength of the surface layer was more than 800 MPa, the three-point flexural strength being higher than that of the first composition-gradient layer by more than 150 MPa. In addition, Examples 1 to 15, which had a composition-gradient layer, were layered bodies having gradations in translucency and color that allow them to give an impression similar to natural teeth.

By contrast, Comparative Example 1, in which the stabilizing element content of the surface layer was greater than the stabilizing element content of the adjacent, first composition-gradient layer, had gradations in translucency and color, but was not a layered body having as high mechanical strength as that of the Examples; the three-point flexural strength of the surface layer was approximately 600 MPa.

It should be noted that the entire contents of the description, claims, drawings and abstract of Japanese Patent Application No. 2022-023814, filed on February 18, 2022, are incorporated herein by reference as a disclosure of the description of the present disclosure.

Reference Signs List

| | |
|---|---|
| 100, 200, 500, 600: | Zirconia sintered body |
| 10: | Surface zirconia layer |
| 20: | Composition-gradient zirconia layer |
| 21: | First composition-gradient zirconia layer |
| 22: | Second composition-gradient zirconia layer |
| 23: | Composition-gradient intermediate zirconia layer |
| 23a: | Third composition-gradient zirconia layer |
| 23b: | Fourth composition-gradient zirconia layer |
| 31: | Horizontal plate |
| 32A, 32B: | Thickness gauge |
| 33: | Sintered body's size |
| 41: | Load |
| 42: | Span |
| 51: | Necking-structured zirconia |
| 61: | Powder composition |
| 62: | Mold |
| 63: | Uniaxial compression |
| 71: | Uniaxially compressed powder (layered body) |
| 72: | Solvent |

**Claims**

1. A layered body comprising a surface layer containing zirconia containing at least one stabilizing element and a composition-gradient layer composed of two or more unit layers,

   the unit layers each containing at least one coloring element and zirconia containing at least one stabilizing element, and
   the composition-gradient layer being constructed as a result of the unit layers being stacked in such a manner that an amount of the stabilizing element in the zirconia containing at least one stabilizing element contained in the composition-gradient layer remains unchanged or decreases from the surface layer toward a surface of the layered body opposite the surface layer, wherein:
   an amount of the stabilizing element in the zirconia containing at least one stabilizing element contained in the surface layer is smaller than an amount of the stabilizing element in the zirconia containing at least one stabilizing element contained in a first composition-gradient layer, which is one of the unit layers constituting the composition-gradient layer and is adjacent to the surface layer.

2. The layered body according to Claim 1, wherein the surface layer further contains at least one coloring element.

3. The layered body according to Claim 1 or 2, wherein the amount of the stabilizing element in the zirconia containing at least one stabilizing element contained in the surface layer is 2.5 mol% or more and 6.0 mol% or less.

4. The layered body according to any one of Claims 1 to 3, wherein a difference between a stabilizing element content of the surface layer and a stabilizing element content of the first composition-gradient layer is 0.2 mol% or more.

5. The layered body according to any one of Claims 1 to 4, wherein the amount of the stabilizing element in the zirconia containing at least one stabilizing element contained in the first composition-gradient layer is 2.5 mol% or more and 6.0 mol% or less.

6. The layered body according to any one of Claims 1 to 5, wherein the stabilizing element is one or more selected from the group of yttrium (Y), calcium (Ca), magnesium (Mg), cerium (Ce), praseodymium (Pr), neodymium (Nd), terbium (Tb), erbium (Er) and ytterbium (Yb).

7. The layered body according to any one of Claims 1 to 6, wherein the coloring element is at least one transition metal element, at least one lanthanoid-series rare earth element or both.

8. The layered body according to any one of Claims 1 to 7, wherein an amount of the coloring element is 0.01% by mass or more and 1.0% by mass or less.

9. The layered body according to any one of Claims 1 to 8, wherein warpage as measured using thickness gauges conforming to JIS B 7524: 2008 is 1.0 mm or less.

10. The layered body according to any one of Claims 1 to 9, wherein the layered body is a sintered body.

11. The layered body according to Claim 10, wherein three-point flexural strength of the surface layer as measured by a method according to JIS R 1601 is 700 MPa or more.

12. The layered body according to any one of Claims 1 to 9, wherein the layered body is a calcined body.

13. A method for manufacturing the layered body according to any one of Claims 1 to 11, the method comprising a step of sintering a green body at 1200°C or above and 1600°C or below,

the green body having a surface powder composition layer containing zirconia containing at least one stabilizing element and a composition-gradient powder composition layer composed of two or more unit powder composition layers,
the unit powder composition layers each containing at least one coloring element and zirconia containing at least one stabilizing element,
the composition-gradient powder composition layer being constructed as a result of the unit powder composition layers being stacked in such a manner that an amount of the stabilizing element in the zirconia containing at least one stabilizing element contained in the composition-gradient powder composition layer remains unchanged or decreases from the surface powder composition layer toward a surface powder composition of the green body opposite the surface powder composition layer, and
an amount of the stabilizing element in the zirconia containing at least one stabilizing element contained in the surface powder composition layer being smaller than an amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

14. A method for manufacturing the layered body according to any one of Claims 1 to 11, the method comprising a step of calcining a green body at 800°C or above and below 1200°C to turn the green body into a calcined body and

a step of sintering the calcined body at 1200°C or above and 1600°C or below,
the green body having a surface powder composition layer containing zirconia containing at least one stabilizing element and a composition-gradient powder composition layer composed of two or more unit powder composition layers,
the unit powder composition layers each containing at least one coloring element and zirconia containing at least one stabilizing element,
the composition-gradient powder composition layer being constructed as a result of the unit powder composition layers being stacked in such a manner that an amount of the stabilizing element in the zirconia containing at least one stabilizing element contained in the composition-gradient powder composition layer remains unchanged or decreases from the surface powder composition layer toward a surface powder composition of the green body opposite the surface powder composition layer, and

an amount of the stabilizing element in the zirconia containing at least one stabilizing element contained in the surface powder composition layer being smaller than an amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

15. A method for manufacturing the layered body according to any one of Claims 1 to 9 or 12, the method comprising a step of calcining a green body at 800°C or above and below 1200°C,

the green body having a surface powder composition layer containing zirconia containing at least one stabilizing element and a composition-gradient powder composition layer composed of two or more unit powder composition layers,

the unit powder composition layers each containing at least one coloring element and zirconia containing at least one stabilizing element,

the composition-gradient powder composition layer being constructed as a result of the unit powder composition layers being stacked in such a manner that an amount of the stabilizing element in the zirconia containing at least one stabilizing element contained in the composition-gradient powder composition layer remains unchanged or decreases from the surface powder composition layer toward a surface powder composition of the green body opposite the surface powder composition layer, and

an amount of the stabilizing element in the zirconia containing at least one stabilizing element contained in the surface powder composition layer being smaller than an amount of the stabilizing element in the zirconia containing at least one stabilizing element contained in a first composition-gradient powder composition layer, which is one of the unit powder composition layers constituting the composition-gradient powder composition layer and is adjacent to the surface powder composition layer.

16. The manufacturing method according to any one of Claims 13 to 15, wherein the surface powder composition layer further contains at least one coloring element.

17. The manufacturing method according to any one of Claims 13 to 16, wherein powder compositions contained in the powder composition layers are powders in a granulated state.

18. A dental material comprising the layered body according to any one of Claims 1 to 12.

FIG. 1

100

Y

X

10
21
22
20

FIG. 2

FIG. 3

FIG. 4

FIG. 5

51

FIG. 6(a)　　　FIG. 6(b)　FIG. 6(c)　　FIG. 6(d)

FIG. 7

## INTERNATIONAL SEARCH REPORT

| International application No. |
| --- |
| **PCT/JP2023/005497** |

**A. CLASSIFICATION OF SUBJECT MATTER**

*C04B 35/488*(2006.01)i; *A61C 5/77*(2017.01)i; *A61C 13/083*(2006.01)i; *A61K 6/78*(2020.01)i; *A61K 6/813*(2020.01)i;
*A61K 6/818*(2020.01)i; *A61K 6/822*(2020.01)i
FI:  C04B35/488; A61K6/818; A61K6/822; A61K6/78; A61K6/813; A61C5/77; A61C13/083

According to International Patent Classification (IPC) or to both national classification and IPC

**B. FIELDS SEARCHED**

Minimum documentation searched (classification system followed by classification symbols)

C04B35/488; A61C5/77; A61C13/083; A61K6/78; A61K6/813; A61K6/818; A61K6/822

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2022/004862 A1 (KURARAY NORITAKE DENTAL INC.) 06 January 2022 (2022-01-06) <br> entire text, all drawings | 1-18 |
| A | JP 2020-147495 A (TOSOH CORP.) 17 September 2020 (2020-09-17) <br> entire text, all drawings | 1-18 |
| A | JP 2020-147494 A (TOSOH CORP.) 17 September 2020 (2020-09-17) <br> entire text, all drawings | 1-18 |
| A | JP 2020-029395 A (TOSOH CORP.) 27 February 2020 (2020-02-27) <br> entire text, all drawings | 1-18 |
| A | JP 2020-142983 A (TOSOH CORP.) 10 September 2020 (2020-09-10) <br> entire text, all drawings | 1-18 |
| P, A | JP 7022875 B1 (KURARAY NORITAKE DENTAL INC.) 18 February 2022 (2022-02-18) <br> entire text, all drawings | 1-18 |

☑ Further documents are listed in the continuation of Box C.    ☑ See patent family annex.

| | |
| --- | --- |
| \* Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **18 April 2023** | **09 May 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

International application No.

**PCT/JP2023/005497**

**C.    DOCUMENTS CONSIDERED TO BE RELEVANT**

| Category* | Citation of document, with indication, where appropriate, of the relevant passages | Relevant to claim No. |
|---|---|---|
| P, A | CN 114180961 A (OPPO GUANGDONG MOBILE COMMUNICATION CO., LTD.) 15 March 2022 (2022-03-15)<br>entire text, all drawings | 1-18 |

Form PCT/ISA/210 (second sheet) (January 2015)

**INTERNATIONAL SEARCH REPORT**
Information on patent family members

International application No.

**PCT/JP2023/005497**

| Patent document cited in search report | | | Publication date (day/month/year) | Patent family member(s) | | | Publication date (day/month/year) |
|---|---|---|---|---|---|---|---|
| WO | 2022/004862 | A1 | 06 January 2022 | JP 2022-60200 A entire text, all drawings | | | |
| JP | 2020-147495 | A | 17 September 2020 | US 2020/0283341 A1 entire text, all drawings EP 3705081 A1 CN 111658552 A | | | |
| JP | 2020-147494 | A | 17 September 2020 | US 2020/0283341 A1 entire text, all drawings EP 3705081 A1 CN 111658552 A | | | |
| JP | 2020-029395 | A | 27 February 2020 | US 2021/0403387 A1 entire text, all drawings EP 3842402 A1 CN 112585103 A | | | |
| JP | 2020-142983 | A | 10 September 2020 | US 2020/0283343 A1 entire text, all drawings EP 3705291 A1 CN 111646793 A | | | |
| JP | 7022875 | B1 | 18 February 2022 | JP 2022-62198 A entire text, all drawings | | | |
| CN | 114180961 | A | 15 March 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160157971
- US 20140328746
- US 20130221554
- JP 2022023814 A